# FASCICULE DE BREVET EUROPEEN

(11) **EP 3 348 249 B1**
(45) Date de publication et mention de la délivrance du brevet: **01.01.2020**
(21) Numéro de dépôt: 17203849.9
(22) Date de dépôt: 27.11.2017
(51) Int. Cl.: A61J 1/20, A61M 5/00, A61M 5/178, A61M 5/31, A61M 39/26, A61J 1/14

(54) **DISPOSITIF POUR LA PRÉPARATION EXTEMPORANÉE D'UNE QUANTITÉ DE FLUIDE STÉRILE**
VORRICHTUNG ZUR BEDARFSMÄSSIGEN ZUBEREITUNG EINER MENGE EINER STERILEN FLÜSSIGKEIT
DEVICE FOR EXTEMPORANEOUS PREPARATION OF AN AMOUNT OF STERILE FLUID

(30) Priorité: 12.01.2017 FR 1750266
(43) Date de publication de la demande: 18.07.2018
(73) Titulaire: Arcadophta, 31100 Toulouse (FR)
(72) Inventeur: REBOUL, Gérard, 31400 TOULOUSE (FR)
(74) Mandataire: Cabinet BARRE LAFORGUE & associés

(56) Documents cités:
- EP-A1- 1 454 650
- WO-A1-95/07720
- WO-A1-2004/078094

## Description

L'invention concerne un dispositif pour la préparation extemporanée d'une quantité de fluide de traitement stérile en vue de son administration à un patient (humain ou animal).

Certaines applications thérapeutiques ou chirurgicales exigent l'administration d'un fluide stérile, dit fluide de traitement, notamment un gaz, par exemple de l'air ou un gaz fluorocarboné (CF₄, C₂F₆, C₃F₈, ...), SF₆, N₂, CO₂, ... Par exemple certains actes de chirurgie ophtalmologiques nécessitent l'administration topique d'un tel gaz de traitement neutre parfaitement stérile dans la cavité oculaire.

Le problème général qui se pose donc dans ce contexte est celui de mettre à la disposition du praticien, une quantité individuelle (c'est-à-dire destinée à un patient unique) de ce fluide à l'état parfaitement stérile, et dans des conditions appropriées à l'administration envisagée.

Dans tout le texte, l'expression « au moins sensiblement » indique, de façon habituelle, qu'une caractéristique structurelle telle qu'une valeur, ou fonctionnelle, ne doit pas être prise comme marquant une discontinuité abrupte, qui n'aurait pas de sens physique, mais couvre non seulement cette structure ou cette fonction, mais également des variations légères de cette structure ou de cette fonction qui produisent, dans le contexte technique considéré, un effet de même nature, sinon de même degré. Par ailleurs les expressions « comportant/comprenant un(e) » sont, sauf indication contraire, synonymes de « comportant/comprenant au moins un(e) », et sont des expressions ouvertes n'excluant pas la présence d'un (ou plusieurs) autre(s) élément(s). Un embout est dit « compatible » avec un autre embout si ces deux embouts peuvent être raccordés l'un à l'autre de façon hermétique, c'est-à-dire en assurant entre eux une communication hermétique de fluide de traitement. Un embout est dit « incompatible » avec un autre embout si ces deux embouts ne peuvent pas être raccordés l'un à l'autre de façon hermétique, c'est-à-dire de façon à assurer entre eux une communication hermétique de fluide de traitement. Un embout est dit « conjugué » d'un autre embout si ces deux embouts présentent des formes permettant leur raccordement hermétique l'un à l'autre, c'est-à-dire de façon à assurer entre une communication hermétique de fluide de traitement. Les termes « hermétique » et « étanche » et leurs dérivés sont présumés être des synonymes parfaits.

WO2004/078094 décrit un dispositif de préparation extemporanée d'une quantité de fluide de traitement stérile en vue de son administration à un patient, comprenant :
- une seringue,
- un réservoir rigide doté d'une valve de sortie rappelée élastiquement axialement vers l'extérieur en position d'obturation et pouvant être repoussée axialement vers l'intérieur en vue de son ouverture pour la libération du fluide de traitement, le réservoir contenant une quantité de fluide de traitement sous pression telle que la quantité totale de fluide de traitement susceptible d'être libérée à pression atmosphérique par le réservoir lors de l'ouverture de la valve du réservoir peut être entièrement contenue dans la seringue,
- un dispositif de raccordement/stérilisation comportant un filtre stérilisant, et adapté pour permettre l'assemblage et le raccordement hermétique du réservoir et de la seringue, et le remplissage de la seringue par une quantité de fluide de traitement stérile par simple rapprochement axial de la seringue et du réservoir.

Après avoir éventuellement dilué le fluide de traitement contenu dans la seringue, par exemple par de l'air atmosphérique si le fluide de traitement est un gaz ophtalmique, le filtre stérilisant est dissocié de la seringue, et une aiguille d'administration est raccordée à l'extrémité de la seringue pour l'administration du fluide de traitement à un patient.

L'extrémité axiale débouchante de la seringue forme un embout tronconique mâle conjugué d'un embout tronconique femelle de la sortie du filtre stérilisant ou de l'aiguille d'administration. De tels embouts tronconiques de raccordement sont en particulier avantageusement des embouts normalisés démontables tronconiques à 6 %, de type conformes à la norme Luer®, de préférence des embouts à verrouillage par vissage de type Luer-lock®. De même, l'entrée du filtre stérilisant présente un embout tronconique mâle conjugué d'un embout tronconique femelle d'un raccord formant un ajutage d'actionnement de la valve de sortie du réservoir. Là encore, le filtre stérilisant étant en général un composant standard du commerce, son embout tronconique mâle de raccordement est un embout normalisé démontable tronconique à 6 % conforme à la norme Luer®.

Ce dispositif donne entière satisfaction. Néanmoins, il a été constaté que certains opérateurs omettent de dissocier le filtre stérilisant de la seringue avant de connecter l'aiguille d'administration à la seringue. Cela est rendu possible par le fait que l'aiguille peut être raccordée à l'entrée du filtre stérilisant, l'embout tronconique femelle de l'aiguille étant conjugué de l'embout tronconique mâle du filtre stérilisant, ces embouts étant compatibles, tous deux conformes à la norme Luer®.

Or, une telle omission fait perdre tout l'intérêt de la stérilisation par filtration, puisque le fluide de traitement ressortant de la seringue via le filtre stérilisant dans l'aiguille d'administration n'est alors plus stérile, et est au contraire contaminé par les micro-organismes précédemment retenus par le filtre lors du remplissage de la seringue.

En outre, il n'est pas non plus exclu que l'entrée du dispositif de raccordement/stérilisation, qui est formée par un ajutage bi-fonctionnel d'actionnement de la valve du réservoir, soit compatible avec une aiguille d'administration. Dans ce cas, le risque existe également une telle aiguille soit directement connectée à l'ajutage, l'opérateur omettant de dissocier le dispositif de raccordement/stérilisation de la seringue, avec les mêmes inconvénients que ceux mentionnés ci-dessus.

En outre, il s'avère que la manipulation de l'ensemble du dispositif nécessite une certaine attention de la part du personnel soignant, notamment pour assembler et aligner axialement correctement la seringue, le dispositif de raccordement/stérilisation et le réservoir, et pour maintenir cet alignement pendant le remplissage de la seringue.

L'invention vise donc à pallier ces inconvénients en proposant un dispositif pour la préparation extemporanée d'une quantité de fluide de traitement -notamment de gaz de traitement- stérile, simple et fiable d'utilisation, évitant toute fuite de fluide lors de l'assemblage et de la préparation -notamment totalement hermétique lors du remplissage de la seringue-, et tout risque de rupture de l'asepsie, et pouvant être lui-même complètement stérilisé sans risque -notamment à l'oxyde d'éthylène-.

L'invention vise en particulier à proposer un tel dispositif qui empêche toute omission de la dissociation du filtre stérilisant ou du dispositif de raccordement/stérilisation de la seringue avant l'administration à un patient du fluide de traitement contenu dans la seringue.

L'invention vise également à proposer un tel dispositif qui facilite et sécurise les manipulations par le personnel soignant.

Pour ce faire, l'invention concerne un dispositif selon la revendication 1.

Ainsi, soit le dispositif de raccordement/stérilisation comprend un embout proximal et cet embout proximal est incompatible avec un tel embout tronconique femelle ; soit il comprend plusieurs embouts proximaux et chaque embout proximal est incompatible avec un tel embout tronconique femelle ; soit il est exempt d'embout proximal.

Par ailleurs, dans un dispositif selon l'invention ladite entrée de fluide du dispositif de raccordement/stérilisation est incompatible avec tout embout tronconique femelle qui serait compatible avec l'embout tronconique mâle de l'extrémité axiale débouchante de la seringue, de sorte que le raccordement hermétique de ladite entrée de fluide du dispositif de raccordement/stérilisation à un tel embout tronconique femelle est impossible.

En effet, ladite entrée de fluide du dispositif de raccordement/stérilisation est formée par ledit embout de l'ajutage dont les formes sont telles qu'il est compatible avec une valve d'un orifice de sortie d'un réservoir, et est incompatible avec un embout tronconique femelle qui serait compatible avec l'embout tronconique mâle de la seringue. Ainsi, ladite entrée de fluide du dispositif de raccordement/stérilisation est en particulier distincte de l'embout tronconique mâle de la seringue, de sorte que le raccordement hermétique d'un embout tronconique femelle conjugué de l'embout tronconique mâle de la seringue à ladite entrée de fluide du dispositif de raccordement/stérilisation est impossible.

En particulier, l'entrée de fluide du dispositif de raccordement/stérilisation est incompatible avec un embout tronconique femelle d'une aiguille d'administration -notamment incompatible avec un embout tronconique femelle à 6% et/ou conforme à la norme Luer®- qui serait conjugué de l'embout tronconique mâle -notamment de l'embout tronconique mâle à 6 % et/ou conforme à la norme Luer®- de la seringue. Ainsi, l'entrée de fluide du dispositif de raccordement/stérilisation est exempt d'embout tronconique mâle, tel qu'un embout tronconique à 6% et/ou conforme à la norme Luer®, similaire à celui de la seringue. Dès lors, il n'est pas possible de raccorder une aiguille d'administration au dispositif de raccordement/stérilisation alors que ce dernier est encore monté sur la seringue. En conséquence, pour raccorder une telle aiguille d'administration à la seringue en vue d'administrer à un patient une quantité de fluide de traitement contenue dans la seringue, l'opérateur doit nécessairement démonter préalablement au moins pour partie le dispositif de raccordement/stérilisation (c'est-à-dire au moins la pièce formant l'entrée du dispositif de raccordement/stérilisation) de la seringue.

Le dispositif de raccordement/stérilisation d'un dispositif selon l'invention peut faire l'objet d'au moins deux variantes principales.

Dans une première variante, le dispositif de raccordement/stérilisation est formé d'une seule et même pièce, d'un seul tenant, faisant simultanément office de dispositif de stérilisation par filtration et de raccord entre la seringue et une valve d'un orifice de sortie d'un réservoir. Dans cette première variante, l'entrée de fluide du dispositif de raccordement/stérilisation est formée dudit ajutage et est incompatible avec un embout tronconique femelle d'une aiguille d'administration, le raccordement hermétique d'une telle aiguille d'administration à l'entrée du dispositif de raccordement/stérilisation est impossible, de sorte que le raccordement d'une telle aiguille d'administration à la seringue nécessite de démonter de la seringue entièrement le dispositif de raccordement/stérilisation, et donc en particulier le dispositif de stérilisation par filtration incorporé dans ce dispositif de raccordement/stérilisation.

Dans une deuxième variante, le dispositif de raccordement/stérilisation comprend au moins un embout proximal interposé entre le dispositif de stérilisation par filtration et ladite entrée de fluide du dispositif de raccordement/stérilisation. Un tel embout proximal est solidaire d'une première pièce (proximale) du dispositif de raccordement/stérilisation et peut être raccordé de façon hermétique mais démontable à un embout distal d'une deuxième pièce (distale) distincte du dispositif de raccordement/stérilisation, distincte de ladite première pièce. Chaque embout proximal étant incompatible avec tout embout tronconique femelle qui serait compatible avec l'embout tronconique mâle de la seringue, le raccordement hermétique d'un embout tronconique femelle d'une aiguille à un tel embout proximal du dispositif de raccordement/stérilisation est impossible. En conséquence, le raccordement d'une aiguille d'administration à la seringue nécessite le démontage de ladite première pièce, et donc en particulier du dispositif de stérilisation par filtration, de la seringue.

En particulier, tout embout proximal du dispositif de raccordement/stérilisation est incompatible avec un embout tronconique femelle d'une aiguille d'administration -notamment incompatible avec un embout tronconique femelle à 6% et/ou conforme à la norme Luer®- qui serait conjugué de l'embout tronconique mâle -notamment de l'embout tronconique mâle à 6 % et/ou conforme à la norme Luer®- de la seringue. Ainsi, en particulier, le dispositif de raccordement/stérilisation est exempt d'embout proximal tronconique mâle, tel qu'un embout tronconique à 6% et/ou conforme à la norme Luer®, similaire à celui de la seringue. Dès lors, il n'est pas possible de raccorder une aiguille d'administration à un embout proximal du dispositif de raccordement/stérilisation alors que ce dernier est au moins pour partie encore monté sur la seringue. En conséquence, pour raccorder une telle aiguille d'administration à la seringue en vue d'administrer à un patient une quantité de fluide de traitement contenue dans la seringue, l'opérateur doit nécessairement démonter préalablement de la seringue au moins toute pièce formant un tel embout proximal, et en particulier le dispositif de stérilisation par filtration.

Ainsi, dans un dispositif selon l'invention, le dispositif de raccordement/stérilisation est totalement exempt de tout embout proximal de raccordement -notamment de tout embout proximal tronconique mâle- qui serait compatible avec un embout tronconique femelle qui lui-même serait compatible avec embout tronconique mâle de la seringue. En particulier, dans certains modes de réalisation préférentiels conformes à l'invention le dispositif de raccordement/stérilisation est totalement exempt de tout embout proximal tronconique mâle interposé entre le dispositif de stérilisation par filtration et ladite entrée de fluide du dispositif de raccordement/stérilisation.

En particulier, avantageusement et selon l'invention le dispositif de stérilisation par filtration présente une entrée de fluide de traitement, le dispositif de raccordement/stérilisation étant adapté pour alimenter cette entrée de fluide de traitement du dispositif de stérilisation par filtration par du fluide de traitement en provenance d'un réservoir raccordé à l'entrée du dispositif de raccordement/stérilisation, et cette entrée de fluide de traitement du dispositif de stérilisation par filtration est incompatible avec un embout tronconique femelle qui lui-même serait compatible avec embout tronconique mâle de la seringue. En particulier, avantageusement et selon l'invention l'entrée du dispositif de stérilisation par filtration est incompatible avec un embout tronconique femelle d'une aiguille d'administration -notamment avec un embout tronconique femelle à 6% et/ou conforme à la norme Luer®- qui serait conjugué de l'embout tronconique mâle -notamment de l'embout tronconique mâle à 6 % et/ou conforme à la norme Luer®- de la seringue. Dans certains modes de réalisation avantageux conformes à l'invention l'entrée du dispositif de stérilisation par filtration est exempte de tout embout tronconique mâle et est distincte d'un embout tronconique mâle. En conséquence, pour raccorder une aiguille d'administration à la seringue, il est nécessaire de démonter préalablement de la seringue au moins le dispositif de stérilisation.

En particulier, dans certains modes de réalisation conformes à l'invention, le dispositif de stérilisation par filtration présente une entrée formant un embout proximal du dispositif de raccordement/stérilisation, cet embout proximal étant adapté pour permettre le raccordement d'un embout distal d'un raccord du dispositif de raccordement/stérilisation, ce raccord s'étendant par rapport au dispositif de stérilisation par filtration à l'opposé de la seringue, et cet embout proximal de l'entrée du dispositif de stérilisation par filtration est incompatible avec tout embout tronconique femelle qui serait compatible avec l'embout tronconique mâle de l'extrémité axiale débouchante de la seringue, de sorte que le raccordement hermétique d'un tel embout proximal de l'entrée du dispositif de stérilisation par filtration à un tel embout tronconique femelle est impossible.

En particulier, avantageusement et selon l'invention l'embout proximal de l'entrée du dispositif de stérilisation par filtration est incompatible avec un embout tronconique femelle d'une aiguille d'administration -notamment avec un embout tronconique femelle à 6% et/ou conforme à la norme Luer®- qui serait conjugué de l'embout tronconique mâle -notamment de l'embout tronconique mâle à 6 % et/ou conforme à la norme Luer®- de la seringue. En conséquence, dans ces modes de réalisation, pour raccorder une aiguille d'administration à la seringue, il est aussi nécessaire de démonter préalablement de la seringue au moins le dispositif de stérilisation.

Ainsi, dans toutes les variantes de l'invention, le raccordement d'une aiguille d'administration à la seringue après remplissage de cette dernière nécessite un démontage préalable au moins du dispositif de stérilisation par filtration. Toute erreur de manipulation est donc ainsi évitée. Dans un dispositif selon l'invention, au moins le dispositif de stérilisation par filtration est donc démontable par rapport à la seringue. En particulier, avantageusement et selon l'invention ladite sortie du dispositif de raccordement/stérilisation d'un dispositif selon l'invention est adaptée pour pouvoir être démontable dudit embout tronconique mâle de l'extrémité axiale débouchante de la seringue.

Dans certains modes de réalisation conformes à l'invention de cette deuxième variante, le dispositif de raccordement/stérilisation comprend au moins un raccord comprenant ledit ajutage, cet ajutage présentant une première extrémité axiale débouchante formant ladite entrée du dispositif de raccordement/stérilisation, et une deuxième extrémité axiale débouchante opposée à la première extrémité, cette deuxième extrémité formant un embout distal compatible avec une entrée du dispositif de stérilisation par filtration de façon à permettre un raccordement hermétique de cet embout distal de la deuxième extrémité de l'ajutage à l'entrée du dispositif de stérilisation par filtration.

Plus particulièrement, dans certains modes de réalisation avantageux de l'invention le dispositif de raccordement/stérilisation est constitué du dispositif de stérilisation par filtration et d'un tel raccord. En particulier, dans certains modes de réalisation de l'invention ce raccord est adapté pour pouvoir être dissocié du dispositif de stérilisation par filtration. Également, dans certains modes de réalisation de l'invention, ce raccord est adapté pour pouvoir être au moins pour partie dissocié du réservoir.

En outre, avantageusement et selon l'invention, ladite première extrémité axiale débouchante de l'ajutage forme un embout bi-fonctionnel d'actionnement d'une valve d'un orifice de sortie d'un réservoir et d'éjection du fluide de traitement hors de ce réservoir. Dans certains modes de réalisation, ledit embout bi-fonctionnel est un embout mâle adapté pour pouvoir coopérer avec une valve de sortie femelle d'un réservoir. Dans d'autres modes de réalisation, ledit embout bi-fonctionnel est un embout femelle adapté pour pouvoir coopérer avec une tige d'une valve de sortie mâle d'un réservoir.

En particulier, dans certains modes de réalisation d'un dispositif selon l'invention l'embout de l'entrée du dispositif de stérilisation par filtration est choisi parmi un embout au moins sensiblement cylindrique mâle et un embout au moins sensiblement cylindrique femelle.

En outre, dans certains modes de réalisation d'un dispositif selon l'invention l'embout de la deuxième extrémité de l'ajutage est choisi parmi un embout au moins sensiblement cylindrique femelle et un embout au moins sensiblement cylindrique mâle.

Par ailleurs, un réservoir de fluide de traitement comprenant un orifice de sortie doté d'une valve est au moins sensiblement cylindrique -notamment au moins sensiblement cylindrique de révolution- et/ou présente un bossage -en particulier un bossage au moins sensiblement cylindrique, notamment au moins sensiblement cylindrique de révolution- entourant cet orifice de sortie et/ou une portée concave -en particulier une portée concave au moins sensiblement cylindrique, notamment au moins sensiblement cylindrique de révolution- entourant cet orifice de sortie, l'orifice de sortie étant centré sur l'axe de symétrie du réservoir et/ou du bossage et/ou de ladite portée concave. En conséquence, un tel réservoir présente au moins une portée -en particulier au moins une portée moins sensiblement cylindrique, notamment au moins sensiblement cylindrique de révolution- entourant l'orifice de sortie. En conséquence, dans certains modes de réalisation conformes à l'invention, le dispositif -notamment ledit raccord- comprend un manchon portant et entourant l'ajutage et présentant une jupe d'extrémité adaptée pour pouvoir coopérer avec une portée entourant un orifice de sortie d'un réservoir pour guider l'assemblage de l'ajutage -notamment dudit raccord- à un tel réservoir.

Une telle jupe d'extrémité du manchon d'un dispositif selon l'invention peut faire l'objet de toutes variantes de formes et dimensions, adaptées à celles de la portée avec laquelle elle est destinée à coopérer. En particulier, dans certains modes de réalisation conformes à l'invention la jupe d'extrémité est adaptée pour pouvoir entourer une portée cylindrique convexe d'un bossage formant un orifice de sortie d'un réservoir. Cette jupe d'extrémité présente alors une paroi interne au moins sensiblement cylindrique -notamment au moins sensiblement cylindrique de révolution-, notamment légèrement tronconique pour faciliter l'assemblage de la jupe d'extrémité autour du bossage et assurer un serrage en fin d'assemblage, par exemple avec un angle de dépouille compris entre 1° et 5°, notamment de l'ordre de 2°. D'autres valeurs sont possibles.

Par exemple, certains réservoirs permettant le conditionnement de gaz sous pression comportent un bossage dans lequel est ménagée une valve d'un orifice de sortie, ce bossage présentant une paroi périphérique globalement au moins sensiblement cylindrique de révolution dont le diamètre extérieur est souvent compris entre 10 mm et 15 mm, typiquement de l'ordre de 13 mm. Pour pouvoir coopérer avec un tel bossage, la jupe d'extrémité du manchon du raccord du dispositif selon l'invention présente avantageusement un diamètre interne supérieur de 0,5 mm à 1 mm, notamment de l'ordre de 0,80 mm, au diamètre extérieur du bossage, c'est-à-dire typiquement de l'ordre de 13,8 mm. D'autres valeurs sont possibles.

Rien n'empêche en outre de prévoir une ou plusieurs nervures parallèles à l'axe de la paroi interne de la jupe d'extrémité et en saillie de sa paroi interne pour faciliter l'assemblage et assurer un serrage du manchon sur ladite portée en fin d'assemblage.

Un même dispositif selon l'invention peut comporter un unique manchon -notamment un unique raccord- de façon à être compatible avec un unique modèle de réservoir, ou au contraire, peut comporter plusieurs manchons -notamment plusieurs raccords- de façon à pouvoir être compatible avec l'un quelconque d'une pluralité de modèles de réservoirs distincts. Dans cette dernière variante, l'opérateur choisit le manchon -notamment le raccord-compatible avec le réservoir qu'il souhaite utiliser avec le dispositif selon l'invention.

Par exemple, un même dispositif selon l'invention comprend une pluralité de dispositifs de raccordement/stérilisation -notamment une pluralité de raccords- dont les manchons présentent des jupes d'extrémité de différents diamètres pour pouvoir coopérer avec des portées de différents diamètres. De même, les différents dispositifs de raccordement/stérilisation -notamment les différents raccords-peuvent comporter des ajutages de formes et/ou dimensions différentes pour pouvoir coopérer avec différentes formes et/ou dimensions de valves de sortie de différents modèles de réservoir.

L'ajutage d'un dispositif selon l'invention peut être fixé rigidement au manchon en étant indissociable de ce dernier, le raccord étant alors adapté pour que lorsque le manchon est assemblé sur un réservoir, la première extrémité de l'ajutage coopère avec la valve de l'orifice de sortie du réservoir pour permettre la sortie du fluide du traitement hors du réservoir.

Néanmoins, dans certains modes de réalisation avantageux selon l'invention, l'ajutage est relié audit manchon par l'intermédiaire d'au moins un lien choisi parmi un lien frangible et un lien déformable, adapté pour permettre un déplacement relatif de l'ajutage par rapport au manchon après assemblage du manchon à un réservoir. En particulier, chaque lien est adapté pour permettre un déplacement de l'ajutage par rapport au manchon après assemblage du manchon à un réservoir, entre une première position dans laquelle le manchon est assemblé au réservoir et l'ajutage n'ouvre pas la valve de l'orifice de sortie du réservoir, et une deuxième position dans laquelle le manchon est assemblé au réservoir et l'ajutage coopère avec la valve de l'orifice de sortie du réservoir pour l'ouvrir. De préférence, chaque lien est frangible (sécable) de telle sorte que le raccord est à usage unique, l'ajutage étant séparé du manchon après utilisation du dispositif selon l'invention, c'est-à-dire lorsque l'ajutage a été placé dans ladite deuxième position.

Dans sa forme la plus simple, un dispositif selon l'invention comporte uniquement une seringue et un dispositif de raccordement/stérilisation, c'est-à-dire en particulier n'incorpore pas un réservoir de fluide de traitement. Un tel dispositif selon l'invention peut être utilisé en combinaison avec un réservoir de fluide de traitement indépendant, pouvant être conditionné et stérilisé (ou non) indépendamment du dispositif selon l'invention.

Néanmoins, dans certains modes de réalisation avantageux un dispositif selon l'invention comprend lui-même un réservoir de fluide de traitement. Un tel réservoir peut être en particulier un réservoir rigide tel qu'une cartouche rigide. Il s'agit par exemple d'une cartouche rigide contenant un gaz ophtalmique sous pression. Il peut être emballé et stérilisé -notamment à l'oxyde d'éthylène- avec la seringue administration et le dispositif de raccordement/stérilisation dans une même enveloppe externe d'emballage stérile du dispositif selon l'invention.

En particulier, un dispositif selon l'invention comprend un réservoir de fluide de traitement comprenant un orifice de sortie doté d'une valve rappelée élastiquement axialement vers l'extérieur en position d'obturation et pouvant être repoussée axialement vers l'intérieur en vue de son ouverture pour la libération du fluide de traitement hors du réservoir.

En outre, dans certains modes de réalisation avantageux conformes à l'invention, le dispositif de raccordement/stérilisation est adapté pour réaliser un assemblage -notamment axial- de la seringue, de ce dispositif de raccordement/stérilisation, et de la valve du réservoir, et pour permettre le remplissage de la seringue, sans fuite de fluide de traitement, par simple rapprochement -notamment par simple rapprochement axial- de la seringue et du réservoir. Dès lors, la manipulation du dispositif selon l'invention aussi bien pour l'assemblage que pour le remplissage de la seringue est particulièrement simple et ergonomique. Elle ne nécessite que deux points d'appui.

En outre, dans ces modes de réalisation, avantageusement et selon l'invention, la valve de l'orifice de sortie du réservoir est une valve femelle et ledit raccord du dispositif de raccordement/stérilisation et la valve de l'orifice de sortie du réservoir sont adaptés pour que, dans ladite deuxième position de l'ajutage par rapport au manchon :
- dans une première course de rapprochement axial relatif, un raccordement hermétique puisse être réalisé entre l'ajutage et la valve de l'orifice de sortie du réservoir, sans actionnement de la valve qui reste fermée,
- dans une deuxième course de rapprochement axial relatif ultérieur au-delà de la première course, la valve soit ouverte par enfoncement de l'ajutage dans la valve.

Un tel dispositif permet en particulier d'éviter tout risque de fuite de fluide au moment même de l'assemblage et du raccordement, puisque la valve reste complètement fermée tant que le raccordement n'est pas complètement hermétique.

En particulier, dans certains modes de réalisation avantageux et selon l'invention, la valve de l'orifice de sortie du réservoir est une valve femelle dotée d'un orifice de distribution, d'un joint périphérique d'étanchéité, et d'une cavité d'actionnement adaptée pour recevoir une extrémité dudit ajutage, qui forme un embout bi-fonctionnel d'actionnement de la valve et d'éjection du fluide de traitement, et est compatible avec la valve femelle du réservoir. Ces modes de réalisation sont particulièrement simples et avantageux, peu coûteux, commodes et fiables à l'utilisation.

Rien n'empêche cependant de prévoir au contraire en variante que la valve de l'orifice de sortie du réservoir soit une valve mâle présentant une tige en saillie axialement à l'extérieur du réservoir, l'ajutage du raccord du dispositif selon l'invention présentant alors un embout femelle conjugué de cette tige en saillie pour réaliser, dans une première course de rapprochement axial relatif, un raccordement hermétique sans ouverture de la valve, puis, dans une deuxième course de rapprochement axial relatif, une ouverture de la valve par enfoncement de la tige de cette dernière.

Par ailleurs, l'invention s'applique à un réservoir contenant une quantité quelconque de fluide de traitement, dès lors que cette quantité est suffisante pour permettre de former au moins une dose individuelle de fluide de traitement dans la seringue. Néanmoins, dans certains modes de réalisation avantageux selon l'invention le réservoir est un réservoir rigide contenant une quantité de fluide de traitement sous pression telle que la quantité totale de fluide de traitement susceptible d'être libérée à pression atmosphérique par le réservoir lors de l'ouverture.

Autrement dit, le cylindre de la seringue est adapté pour pouvoir contenir toute la quantité de fluide de traitement (quantité individuelle) pouvant être libérée par le réservoir rigide à la pression atmosphérique.

Le fait d'utiliser un réservoir rigide permet notamment en pratique de réaliser une stérilisation parfaite de l'ensemble du dispositif. En effet, un tel réservoir rigide peut être notamment réalisé en une matière choisie parmi les matériaux métalliques, les verres, les matériaux polymériques synthétiques rigides formant une barrière parfaite au fluide de stérilisation tel que l'oxyde d'éthylène. En outre, un tel réservoir rigide peut renfermer le fluide de traitement sans aucun risque de contamination de ce fluide de traitement.

Par ailleurs, un tel réservoir rigide pressurisé qui ne contient qu'une quantité individuelle de fluide de traitement, c'est-à-dire une dose destinée à une administration unique, permet de faciliter l'opération de remplissage de la seringue, y compris dans le cas d'un gaz de traitement, en produisant un déplacement spontané du piston par équilibrage des pressions tout en évitant à la fois l'expulsion intempestive du piston hors du cylindre, l'introduction d'une quantité insuffisante, et l'emploi d'une valve doseuse, généralement inefficace ou imprécise dans le cas d'un gaz, et en tout cas très onéreuse. Ainsi, le remplissage de la seringue s'effectue automatiquement sans nécessiter aucun contrôle visuel du dosage de la quantité introduite dans la seringue de la part de l'utilisateur y compris dans le cas où le fluide de traitement est compressible. En pratique, il suffit de prévoir que le volume maximum du cylindre de la seringue (piston en position extrême éloigné de l'extrémité de distribution de la seringue) soit supérieur à la quantité de fluide de traitement (destinée à une administration unique) pouvant être libérée par le réservoir à la pression atmosphérique, cette quantité pouvant néanmoins être elle-même supérieure ou égale à la quantité posologique requise pour le traitement d'un seul et même patient (l'utilisateur (praticien) pouvant ensuite aisément refaire sortir le surplus de fluide de traitement hors du cylindre en repoussant le piston avant l'administration).

Par ailleurs, le dispositif selon ces modes de réalisation de l'invention étant à usage unique et doté d'un dispositif de stérilisation par filtration du fluide de traitement pénétrant dans la seringue elle-même préalablement stérilisée, le caractère stérile de la quantité de fluide de traitement contenu dans la seringue est garanti. En particulier, on minimise les risques de contamination -notamment au niveau de la valve de sortie du réservoir- par des micro-organismes pathogènes pouvant résulter d'un usage multiple du même réservoir de fluide. Et même en cas d'infection des parties extérieures de la valve de sortie, le fluide est stérilisé par le dispositif de stérilisation avant d'entrer dans la seringue. En outre, avec des réservoirs à quantité individuelle et usage unique, il est possible de réaliser le suivi des quantités de fluide administrées (« traçabilité »).

Par ailleurs, dans certains modes de réalisation préférentiels d'un dispositif selon l'invention le dispositif de stérilisation par filtration présente une sortie formant ladite sortie du dispositif de raccordement/stérilisation. Ainsi, dans ces modes de réalisation, le dispositif de stérilisation par filtration est directement raccordé à la seringue, et présente à cet effet un embout tronconique femelle de raccordement conjugué de l'embout tronconique mâle de la seringue. Ces embouts sont de préférence des embouts démontables, par exemple de type Luer®.Rien n'empêche de prévoir en variante qu'un raccord démontable soit interposé entre la seringue et le dispositif de stérilisation par filtration, notamment pour pouvoir utiliser un dispositif de stérilisation par filtration ne présentant pas d'embout tronconique femelle de raccordement compatible avec l'embout tronconique mâle de la seringue. Également, tel raccord peut par exemple être doté d'un robinet d'arrêt permettant notamment d'éviter toute entrée ou sortie de fluide hors de la seringue lors de la mise en place de l'aiguille d'administration sur la seringue.

Un dispositif selon certains modes de réalisation de l'invention n'incorpore pas lui-même une aiguille d'administration. Un tel dispositif selon l'invention peut être utilisé en combinaison avec une aiguille d'administration indépendante du dispositif selon l'invention, une telle aiguille pouvant être conditionnée et stérilisée indépendamment du dispositif selon l'invention. En effet, les aiguilles du commerce sont dotées d'un embout de raccordement normalisé démontable tronconique femelle, notamment à 6% et/ou conforme à la norme Luer®.

Néanmoins, dans certains modes de réalisation avantageux un dispositif selon l'invention comprend lui-même une aiguille stérile comprenant un embout tronconique femelle conjugué de l'embout tronconique mâle de la seringue permettant le raccordement hermétique de l'aiguille à l'extrémité de la seringue.

Une telle aiguille permet notamment l'administration à un patient du fluide de traitement contenu dans la seringue. Elle peut être emballée et stérilisée -notamment à l'oxyde d'éthylène-, éventuellement avec un pré-emballage de cette aiguille, avec la seringue administration et le dispositif de raccordement/stérilisation, voire avec un réservoir, dans une même enveloppe externe d'emballage stérile du dispositif selon l'invention. L'embout tronconique femelle de l'aiguille stérile est en particulier incompatible avec l'entrée du dispositif de raccordement/stérilisation.

Par ailleurs, un dispositif selon certains modes de réalisation de l'invention peut également avantageusement présenter tout ou partie des caractéristiques mentionnées par WO2004/078094.

En particulier, le réservoir comprend avantageusement un opercule de fermeture hermétique de la valve adapté pour empêcher toute pénétration de fluide de stérilisation dans la valve et dans le réservoir, et pour pouvoir être brisé par introduction de l'ajutage bi-fonctionnel dans l'orifice de la valve. Cet opercule, qui comprend par exemple une pellicule d'aluminium, permet aussi d'éviter tout contact du fluide de traitement et/ou de la valve du réservoir avec le fluide de stérilisation -notamment l'oxyde d'éthylène-, qui est toxique, utilisé pour stériliser l'ensemble du dispositif selon l'invention.

Avantageusement, le dispositif selon l'invention comprend aussi une étiquette -notamment sous forme de bracelet- destinée à être associée au patient, pouvant porter une référence identifiant le réservoir de fluide de traitement utilisé. Ce bracelet permet de signaler au personnel soignant le fait que le patient a été traité par le fluide de traitement, de sorte que des précautions appropriées peuvent être prises pour les soins et traitements ultérieurs. Cela est particulièrement avantageux et important dans le cas d'un gaz de traitement.

Le dispositif selon l'invention comprend en outre avantageusement une enveloppe externe d'emballage stérile renfermant l'intégralité des éléments constitutifs du dispositif à l'état stérile. Il se présente ainsi en un paquet unique emballé stérile prêt à l'emploi sur le site d'administration.

Avantageusement et selon l'invention, la seringue et le dispositif de stérilisation par filtration se présentent préalablement assemblés à l'état stérile dans l'enveloppe. En particulier, la seringue et le dispositif de raccordement/stérilisation sont préalablement assemblés et se présentent stériles et assemblés dans l'enveloppe. De la sorte, on garantit que la cavité interne de la seringue recevant le fluide de traitement ne puisse jamais venir être contaminée, même à l'ouverture de l'enveloppe, grâce au dispositif de stérilisation qui empêche toute pénétration de germe dans cette cavité. En outre, même si la sortie de la valve du réservoir et/ou l'opercule qu'elle porte est infecté(e), le fluide de traitement pénétrant dans la seringue est stérilisé.

Dans un procédé de préparation extemporanée d'une quantité individuelle de fluide de traitement stérile pouvant être compressible -notamment un gaz de traitement- en vue de son administration à un patient, à l'aide d'un dispositif selon l'invention, on assemble la seringue, le dispositif de raccordement/stérilisation et la valve du réservoir, de façon à réaliser entre eux une communication isolée hermétiquement de l'extérieur, puis on rapproche -notamment axialement- la seringue et le réservoir de façon à rompre chaque lien reliant l'ajutage au manchon du raccord, à déplacer l'ajutage et à ouvrir la valve du réservoir, une dose prédéterminée de fluide de traitement stérile étant alors introduite dans la seringue, par équilibrage des pressions. Le réservoir est ensuite séparé de la seringue, l'ajutage étant séparé de la valve du réservoir. Le fluide de traitement peut ensuite être utilisé pur ou dilué. Dans ce dernier cas, lorsque le fluide de traitement est un gaz de traitement, on dilue le fluide de traitement dans la seringue en :
- repoussant le piston pour éjecter le surplus de fluide de traitement hors de la seringue,
- tirant le piston pour introduire dans la seringue une quantité d'air atmosphérique de dilution via le dispositif de stérilisation par filtration.

On dissocie ensuite (après préparation de la quantité de fluide de traitement à administrer dans la seringue) le dispositif de stérilisation par filtration de la seringue à laquelle on raccorde une aiguille d'administration. Avec un dispositif selon l'invention, il n'est pas possible de raccorder une aiguille d'administration directement au dispositif de stérilisation par filtration, de sorte qu'il est nécessaire de séparer ce dispositif de stérilisation par filtration de la seringue avant de pouvoir raccorder une aiguille d'administration. On peut ensuite utiliser la seringue avec l'aiguille pour l'administration du fluide de traitement au patient.

Le dispositif selon l'invention est particulièrement simple, fiable, et garantit une grande sécurité d'utilisation, sans risque de fuite, d'accident ni de contamination, y compris dans le cas d'un fluide de traitement compressible. Il permet aussi de garantir le suivi des doses de fluide administrées (« traçabilité »).

L'invention concerne aussi un dispositif caractérisé en combinaison ou non, par tout ou partie des caractéristiques mentionnées ci-dessus ou ci-après. Quelle que soit la présentation formelle qui en est donnée, sauf indication contraire explicite, les différentes caractéristiques mentionnées ci-dessus ou ci-après ne doivent pas être considérées comme étroitement ou inextricablement liées entre elles, l'invention pouvant concerner l'une seulement de ces caractéristiques structurelles ou fonctionnelles, ou une partie seulement de ces caractéristiques structurelles ou fonctionnelles, ou une partie seulement de l'une de ces caractéristiques structurelles ou fonctionnelles, ou encore tout groupement, combinaison ou juxtaposition de tout ou partie de ces caractéristiques structurelles ou fonctionnelles..

D'autres buts, caractéristiques et avantages de l'invention apparaîtront à la lecture de la description suivante d'un de ses modes de réalisation donné à titre d'exemple non limitatif, et qui se réfère aux figures annexées dans lesquelles :
- la figure 1 est un schéma en perspective illustrant un exemple de dispositif selon l'invention,
- la figure 2 est une vue schématique en perspective éclatée illustrant un raccord, un dispositif de stérilisation par filtration et une seringue d'un dispositif selon l'invention,
- la figure 3 est une vue schématique par une première extrémité axiale d'un raccord d'un dispositif selon l'invention,
- la figure 4 est une vue selon la ligne IV-IV de la figure 3,
- les figures 5 à 10 sont des vues schématiques en coupe illustrant respectivement différentes étapes successives d'un procédé d'utilisation d'un dispositif selon l'invention,
- la figure 11 est une vue partielle en coupe d'un exemple de réalisation d'un dispositif selon l'invention après assemblage au réservoir de la seringue avec le dispositif de raccordement/stérilisation,
- la figure 12 est une vue similaire à la figure 11 illustrant le dispositif selon l'invention au cours du remplissage de la seringue,
- la figure 13 est une vue schématique en perspective du dispositif selon l'invention après remplissage de la seringue et séparation du réservoir.

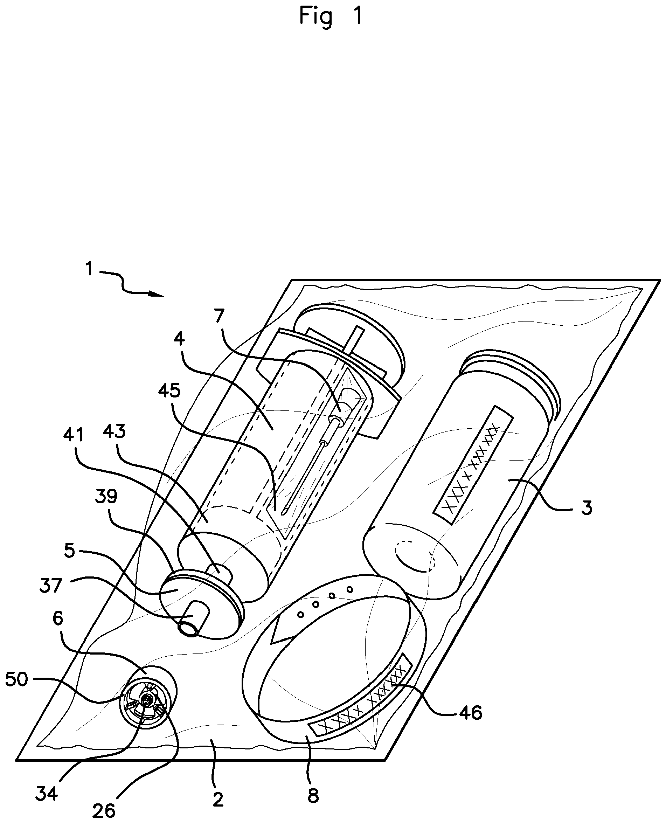

Le dispositif 1 selon l'invention représenté figure 1 se présente à l'état conditionné stérile, prêt à l'emploi, et comprend une enveloppe externe 2 d'emballage stérile définissant une enceinte close hermétique renfermant les différents éléments constitutifs du dispositif 1, à savoir :
- un réservoir rigide 3 contenant une quantité de fluide de traitement -notamment un gaz de traitement- sous pression,
- une seringue d'administration 4,
- un dispositif 5 de stérilisation par filtration,
- un raccord 6 destiné à être interposé entre le dispositif 5 de stérilisation par filtration et le réservoir 3,
- une aiguille d'administration 7,
- un bracelet 8 destiné à être porté par le patient.

Après emballage dans l'enveloppe externe 2, le dispositif est stérilisé à l'oxyde d'éthylène, de sorte que tous ses éléments constitutifs sont stériles. En outre, tous ses éléments constitutifs sont de type jetable à usage unique.

Le dispositif selon l'invention tel que représenté sur les figures présente les caractéristiques générales décrites dans WO 2004/078094, seules les caractéristiques propres à la présente invention étant décrites ci-après en détail.

Le réservoir est une cartouche 3 qui comprend un boîtier externe rigide 9, qui peut être métallique -notamment en aluminium ou en fer blanc- ou en tout autre matière rigide telle que les verres et les polymères rigides définissant une ouverture d'extrémité refermée par une valve 10 de sortie portant une poche 11 s'étendant à l'intérieur du boîtier 9 et associée avec son enceinte interne en communication de fluide avec la valve 10.

Le réservoir 3 comprend une paroi rigide d'obturation 12 fixée à l'extrémité du boîtier 9 de façon hermétique par l'intermédiaire d'un joint d'étanchéité, et présentant un orifice 14 circulaire central de distribution de la valve 10. Cet orifice 14 est ménagé à l'extrémité axiale d'un bossage 52 cylindrique de révolution formé par la paroi 12 et contenant au moins pour partie la valve 10.

La valve 10 comprend une soupape 19 rappelée élastiquement axialement en position d'obturation par un ressort de compression 21, et un joint d'étanchéité 17 autour de son orifice 14. La soupape 19 est adaptée pour recevoir une première extrémité axiale 34 débouchante d'un ajutage 26 rigide d'éjection du fluide de traitement et d'actionnement de la valve 10. Cet ajutage 26 est formé d'une portion tubulaire creuse symétrique de révolution présentant, à sa première extrémité axiale 34, un diamètre externe correspondant à celui du joint d'étanchéité 17. La première extrémité axiale 34 de l'ajutage 26 est bi-fonctionnelle en ce sens qu'elle peut exercer une première fonction consistant à actionner la soupape 19 et la valve 10 pour l'ouvrir, et une deuxième fonction consistant à réaliser un passage d'éjection du fluide de traitement hors de la valve 10 et du réservoir 3.

La poche 11 définit, d'une part, une enceinte interne 29 qui contient le fluide de traitement et est en communication avec la soupape 19, et, d'autre part, entre cette poche 11 et la paroi rigide externe du boîtier 9, une enceinte intermédiaire 30 renfermant une quantité appropriée de gaz pousseur pressurisé à l'état gazeux. Ce gaz pousseur est de préférence un gaz physiologiquement acceptable, compatible avec le fluide de traitement et avec le procédé de stérilisation, notamment avec l'oxyde d'éthylène. De la sorte, on évite, d'une part, toute complication en cas de diffusion accidentelle du gaz pousseur dans le fluide de traitement, et, d'autre part, tout risque d'accident au cours de l'étape de stérilisation à l'oxyde d'éthylène.

La quantité de gaz pousseur contenue dans l'enceinte intermédiaire 30 est adaptée pour pressuriser le fluide de traitement dans l'enceinte 29 de la poche 11 à une pression relative appropriée. Avantageusement et selon l'invention, la quantité de gaz pousseur est adaptée pour maintenir dans la poche 11 une surpression de fluide de traitement (par rapport à la pression atmosphérique) inférieure à 2 000 hPa -notamment de l'ordre de 600 à 1 800 hPa-. Cette surpression est de valeur relativement faible mais suffisante pour le remplissage spontané de la seringue (le piston étant mû par la pression du fluide de traitement expulsé de la valve), sans détendeur, ni robinet d'arrêt. La poche 11 est par ailleurs d'un volume correspondant à celui de la quantité de fluide de traitement devant être délivrée par le réservoir 3, inférieur ou égal au volume maximum du cylindre de la seringue 4. En pratique, ce volume de fluide de traitement est par exemple typiquement compris entre 10ml et 100ml -notamment de l'ordre de 40ml-.

L'invention est plus particulièrement avantageusement applicable au cas où le fluide de traitement est un gaz de traitement. Ce gaz de traitement peut être notamment choisi parmi les gaz fluorocarbonés (CF₄, C₂F₆, C₃F₈ ...), SF₆, N₂, C0₂ ou de l'air. Le gaz pousseur peut être de même nature que le fluide de traitement.

L'orifice 14 de distribution de la paroi 12 d'obturation de la valve 10 peut être refermé de façon hermétique avant utilisation par un opercule de fermeture hermétique de cet orifice 14. Cet opercule est collé hermétiquement à l'extérieur de la paroi 12 et est adapté pour empêcher toute pénétration de fluide de stérilisation dans la valve 10, c'est-à-dire dans la poche 11. Pour ce faire, dans le cas notamment où on utilise l'oxyde d'éthylène à titre de gaz de stérilisation, l'opercule comprend par exemple une feuille d'aluminium. Cet opercule est néanmoins suffisamment fin pour pouvoir être brisé par introduction de l'ajutage bi-fonctionnel 26 dans l'orifice 14 de la valve 10 en vue du remplissage d'une seringue. L'opercule est en matériau non élastique de façon à permettre une rupture facile de cet opercule par l'ajutage 26. Il doit néanmoins être suffisamment épais pour offrir une résistance imposant l'exercice d'un effort axial au-delà d'une valeur seuil pour entraîner sa rupture. De la sorte, on évite toute rupture involontaire de l'opercule, et donc tout actionnement intempestif de la valve 10. L'opercule fait aussi office de garantie d'inviolabilité.

Avant utilisation, le réservoir 3 contient donc une quantité de fluide de traitement à l'intérieur de l'enceinte 29 de la poche 11 sous une pression relative de l'ordre de 600 à 1 800 hPa, maintenue par une pression relative identique de gaz pousseur dans l'enceinte intermédiaire 30.

Le raccord 6 est en une matière synthétique pouvant être stérilisée, par exemple en ABS. Il comprend ledit ajutage 26 dont la première extrémité axiale 34 est compatible avec la valve 10 femelle du réservoir 3, et, à l'opposé de la première extrémité axiale 34, une deuxième extrémité axiale 36 débouchante formant un embout distal de raccordement compatible avec une entrée 38 du dispositif 5 de stérilisation par filtration. L'ajutage 26 est creux de façon à former un passage axial de fluide à travers l'ajutage 26 et sur toute sa longueur entre ses deux extrémités axiales 34, 36.

La première extrémité axiale 34 de l'ajutage 26 formé par ce raccord 6 doit présenter des dimensions compatibles avec celles de la valve 10, c'est-à-dire avec celles de l'orifice 14 de distribution, du joint d'étanchéité 17, et de la soupape 19. Il doit être suffisamment rigide pour permettre de briser l'opercule 31 et repousser la soupape 19 à l'encontre du ressort 21 axialement.

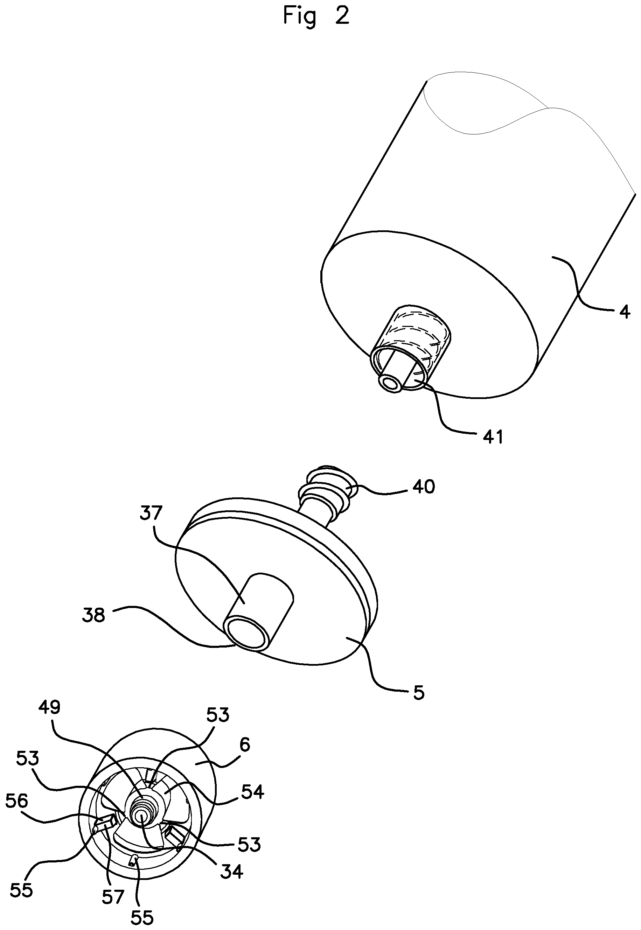

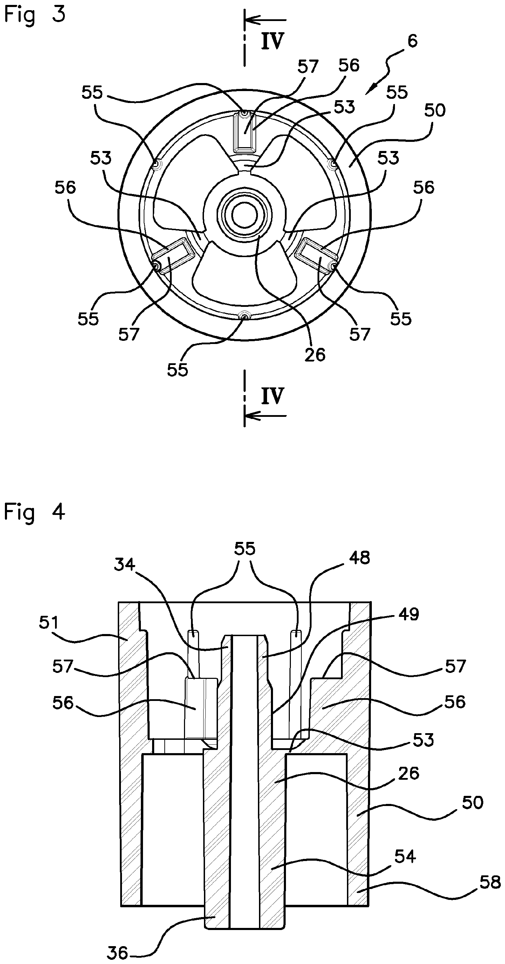

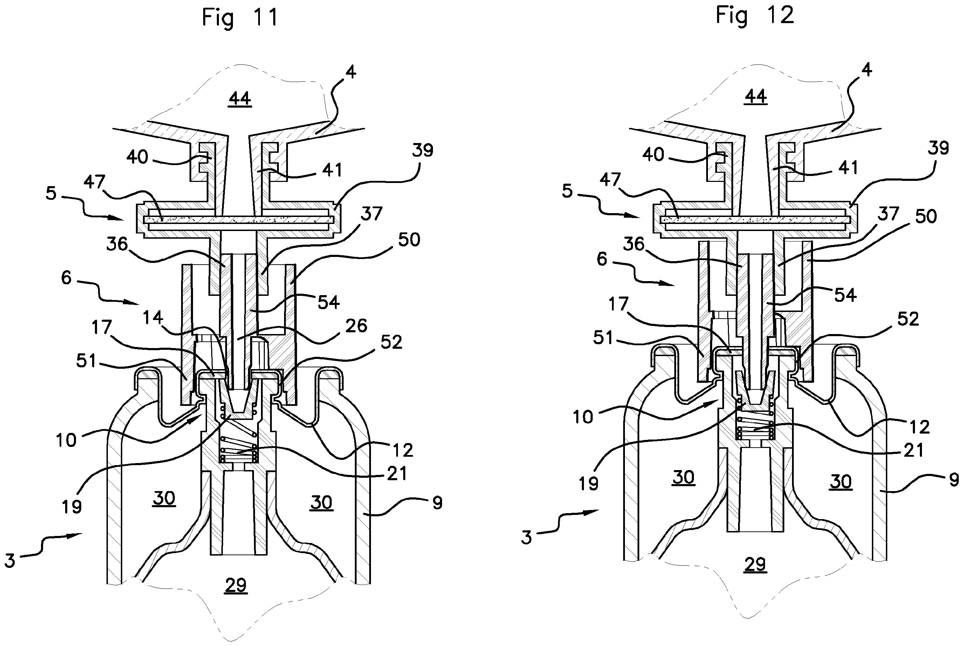

Comme on le voit figures 2, 4, 11 et 12 notamment, la première extrémité axiale 34 de l'ajutage 26 présente un premier tronçon 48 cylindrique de révolution de diamètre inférieur au diamètre interne du joint d'étanchéité 17 de façon à pouvoir facilement pénétrer à l'intérieur de l'orifice 14 de la valve 10, et un deuxième tronçon 49 cylindrique de révolution de diamètre légèrement supérieur au diamètre interne du joint d'étanchéité 17, de sorte que lorsque l'on introduit la première extrémité axiale 34 de l'ajutage 26 dans l'orifice 14, le joint d'étanchéité 17 vient en contact de la paroi externe du deuxième tronçon 49 en assurant l'étanchéité au fluide de traitement, notamment au gaz de traitement.

Le deuxième tronçon 49 de l'ajutage 26 est prolongé par un corps cylindrique 54 de l'ajutage 26 qui s'étend jusqu'à former la deuxième extrémité axiale 36 de l'ajutage 26. Ce corps cylindrique 54 présente un diamètre supérieur à celui du deuxième tronçon 49 et à celui de l'orifice 14 de la valve 10, de façon à ne pas pouvoir pénétrer dans la valve 10.

Le raccord 6 comporte également un manchon 50 cylindrique de révolution portant et entourant l'ajutage 26 et présentant une jupe 51 d'extrémité adaptée pour pouvoir entourer la paroi cylindrique du bossage 52 formé par la paroi 12 d'extrémité du réservoir 3. L'ajutage 26 s'étend à l'intérieur du manchon 50 avec sa première extrémité axiale 34 orientée du même côté que la jupe 51 du manchon 50. L'ajutage 26 et le manchon 50 sont formés d'une seule et même pièce issue de moulage, et l'ajutage 26 est relié au manchon 50 par l'intermédiaire de trois liens radiaux 53 frangibles (sécables) s'étendant, dans l'exemple représenté, à partir de l'épaulement de jonction du deuxième tronçon 49 avec le corps cylindrique 54 de l'ajutage 26. De tels liens frangibles 53 sont formés par une portion de faible largeur et de faible épaisseur susceptible de se rompre lorsque l'ajutage 26 est déplacé axialement par rapport au manchon 50 cylindrique.

Par exemple, chaque lien frangible 53 est formé d'un rayon s'étendant entre la paroi interne du manchon 50 et l'ajutage 26, en forme générale de triangle dont la largeur est décroissante depuis le manchon 50 jusqu'à l'ajutage 26, l'ajutage 26 étant relié à la pointe (extrémité radiale de moindre largeur) du triangle formé par ce rayon. De même, l'épaisseur du rayon formant le lien frangible 53 peut être réduite au niveau de sa jonction à l'ajutage 26 pour faciliter sa rupture sous l'effet d'un effort axial de déplacement de l'ajutage 26 par rapport au manchon 50.

Le manchon 50 présente également, sous chaque lien frangible 53, une nervure 56 de blocage s'étendant en direction de la jupe 51 parallèlement à l'axe du manchon 50, en présentant un épaulement 57 de blocage adapté pour venir en butée contre le bossage 52 du réservoir 3 en fin d'assemblage du manchon 50 sur ce bossage 52. Les nervures 56 de blocage permettent d'une part de contrôler la position axiale et l'alignement du manchon 50 par rapport à la valve 10 du réservoir 3 en fin d'assemblage, d'autre part de rigidifier les liens 53 frangibles en empêchant leur flexion lors du déplacement axial de l'ajutage 26 par rapport au manchon 50, de façon à faciliter et assurer leur rupture au niveau de leur extrémité de jonction à l'ajutage 26.

La jupe 51 d'extrémité du manchon 50 présente également des nervures 55 de serrage en saillie légèrement en surépaisseur vers l'intérieur par rapport à sa paroi interne cylindrique, ces nervures 55 de serrage s'étendant parallèlement à l'axe du manchon 50, uniformément réparties autour de cet axe, pour faciliter l'assemblage et assurer un certain serrage du manchon 50 sur la paroi cylindrique du bossage 52.

Le manchon 50 du raccord 6 facilite l'assemblage de la seringue 4 sur le réservoir 3, et le centrage de l'ajutage 26 par rapport à l'orifice 14 de la valve 10. Après assemblage du manchon 50 sur le bossage 52 du réservoir 3, les épaulements 57 de blocage du manchon 50 étant en butée axialement contre le bossage 52, un déplacement axial relatif de l'ajutage 26 par rapport au manchon 50 a pour effet de rompre les liens 53 frangible et de séparer de façon irréversible l'ajutage 26 du manchon 50.

La longueur axiale de la première extrémité axiale 34 de l'ajutage 26 est de préférence adaptée (suffisamment faible) pour que le contact hermétique puisse être établi entre la paroi externe du deuxième tronçon 49 et le joint d'étanchéité 17 lors d'une première course de rapprochement axial et d'introduction dans l'orifice 14, sans que cette extrémité 34 ne repousse la soupape 19, c'est-à-dire sans ouverture de la valve 10. Mais la longueur axiale du deuxième tronçon de la première extrémité axiale 34 de l'ajutage 26 est adaptée pour que dans une deuxième course de rapprochement axial au-delà de ladite première course (l'étanchéité étant donc établie), l'extrémité 34 de l'ajutage 26 vienne au contact de la soupape 19 pour entraîner son déplacement et l'ouverture de la valve 10.

Il est à noter que la présence éventuelle d'un opercule a aussi pour effet d'obliger l'utilisateur à exercer un effort axial de valeur suffisante, pour que la manœuvre d'introduction de l'ajutage 26 dans la valve 10 jusqu'à son actionnement, se déroule correctement, quasi-instantanément et sans risque de fuite.

Lorsque l'ajutage 26 est introduit à l'intérieur de la valve 10 et que la soupape 19 est repoussée axialement, le fluide de traitement dans la poche 11 peut circuler à travers la soupape 19 et pénétrer à l'intérieur de l'ajutage 26 pour s'écouler axialement dans l'ajutage 26 vers l'extérieur du réservoir 3. La valve 10 de sortie ainsi formée est donc une valve rappelée élastiquement axialement vers l'extérieur en position d'obturation par le ressort 21, et pouvant être repoussée manuellement axialement vers l'intérieur en vue de son ouverture pour la libération du fluide de traitement et ce, grâce à l'ajutage 26 bi-fonctionnel d'éjection du fluide de traitement et d'actionnement de la valve 10.

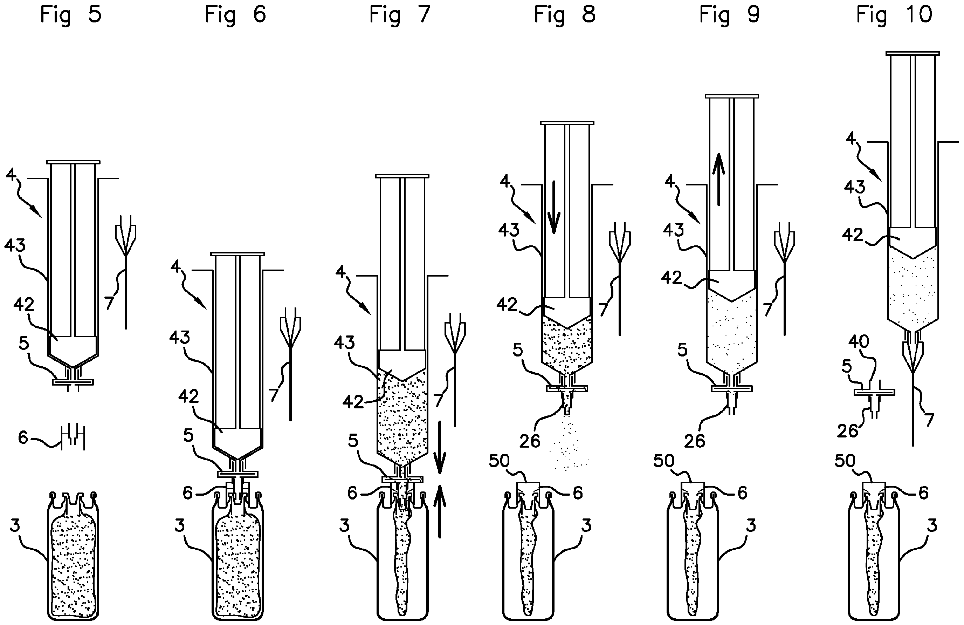

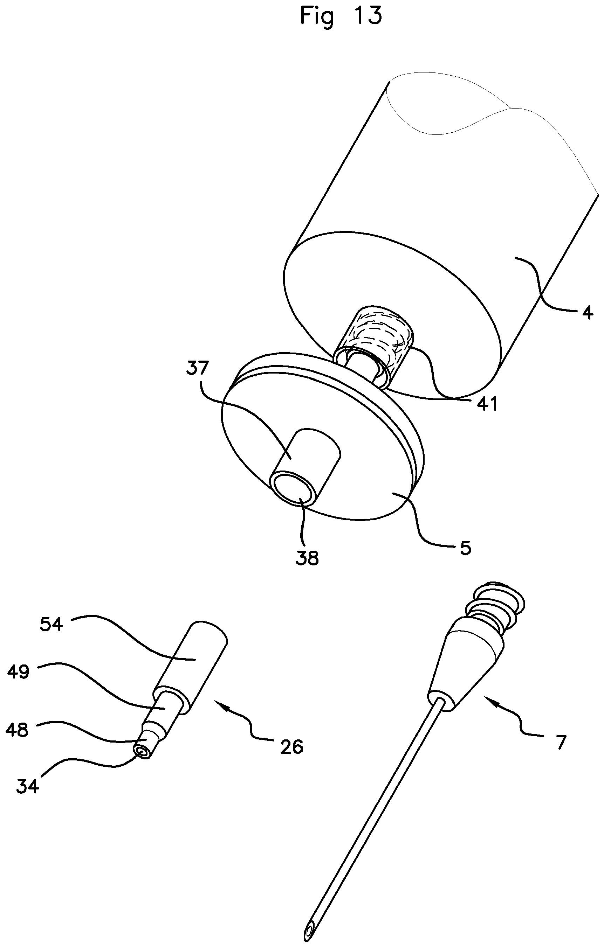

La première extrémité axiale 34 de l'ajutage 26 forme ainsi une entrée du dispositif 5, 6 de raccordement/stérilisation et un embout de raccordement à la valve 10 du réservoir, et cet embout de raccordement n'est pas lui-même compatible avec un embout tronconique femelle d'une aiguille d'administration. Cet embout de raccordement est notamment incompatible avec un embout tronconique femelle à 6 % conforme à la norme Luer®, en particulier conforme à la norme Luer-Lok®. Ainsi, une aiguille d'administration dotée d'un tel embout tronconique femelle, telle que l'aiguille 7 du dispositif selon l'invention fournie dans l'enveloppe 2, ne peut pas être raccordée à la première extrémité axiale 34 de l'ajutage 26 (cf. notamment figures 9 et 13).

Le dispositif 5 de stérilisation par filtration comprend un carter clos hermétique rigide 39, par exemple en PVC, renfermant un filtre 47 -notamment un microfiltre- formé d'une membrane dont les pores ont une dimension de l'ordre de 0,2µm, et sont aptes à retenir les micro-organismes pathogènes. Ce carter 39 présente une entrée 38 de fluide et une sortie 40 de fluide. L'entrée 38 du carter 39 forme l'entrée de fluide du dispositif 5 de stérilisation par filtration. La sortie 40 du carter 39 forme la sortie de fluide du dispositif 5 de stérilisation par filtration.

De préférence, l'extrémité de distribution 41 de la seringue 4 forme un embout tronconique mâle démontable à verrouillage par vissage, notamment à 6 % conforme à la norme Luer-Lok® (ISO 594, EN 1707:1996 et 20594:1993). De la sorte, cette extrémité 41 de la seringue 4 est compatible avec un embout tronconique femelle démontable à verrouillage par vissage -notamment à 6 % conforme à la norme Luer-Lok®- d'une aiguille d'administration. En outre, la sortie de fluide 40 du dispositif 5 de stérilisation par filtration forme un embout tronconique femelle démontable à verrouillage par vissage conjugué de l'embout tronconique mâle à verrouillage de l'extrémité 41 de la seringue 4, cet embout tronconique femelle étant notamment un embout tronconique à 6 % conforme à la norme Luer-Lok®. De la sorte, la sortie 40 du dispositif 5 de stérilisation par filtration peut être directement et hermétiquement raccordée à l'extrémité 41 de la seringue 4, et le dispositif 5 de stérilisation par filtration peut être dissocié de la seringue 4 après utilisation.

Par contre, l'entrée 38 du dispositif 5 de stérilisation par filtration forme un embout 37 proximal de raccordement compatible avec l'embout distal formé par la deuxième extrémité axiale 36 de l'ajutage 26, mais incompatible avec un embout tronconique femelle d'une aiguille d'administration, notamment incompatible avec un embout tronconique femelle à 6 % conforme à la norme Luer®, en particulier conforme à la norme Luer-Lok®. Ainsi, une aiguille d'administration dotée d'un tel embout tronconique femelle, telle que l'aiguille 7 du dispositif selon l'invention fournie dans l'enveloppe 2, ne peut pas être raccordée à l'entrée 38 du dispositif 5 de stérilisation par filtration.

Dans l'exemple représenté, l'embout 37 de l'entrée 38 du dispositif 5 de stérilisation par filtration est un embout au moins sensiblement cylindrique femelle conjugué du corps 54 cylindrique de l'ajutage 26 formant, à la deuxième extrémité axiale 36 de cette ajutage 26, un embout de raccordement au moins sensiblement cylindrique mâle. Toute autre variante de réalisation est possible dès lors que l'embout 37 de l'entrée 38 du dispositif 5 de stérilisation par filtration est compatible avec la deuxième extrémité axiale 36 de l'ajutage 26, et que ni cet embout 37 de raccordement, ni la première extrémité axiale 34 de l'ajutage 26, ne sont compatibles avec un embout tronconique femelle d'une aiguille d'administration, notamment avec un embout tronconique femelle à 6 % conforme à la norme Luer®, en particulier conforme à la norme Luer-Lok®. L'embout 37 de l'entrée 38 du dispositif 5 de stérilisation par filtration peut en particulier être un embout au moins sensiblement cylindrique mâle, ou un embout tronconique femelle, conforme ou non à la norme Luer®.Dès lors, avec un dispositif selon l'invention, il n'est pas possible de raccorder directement une telle aiguille d'administration à l'ajutage 26 ou au dispositif 5 de stérilisation par filtration, ces organes devant impérativement être dissociés de la seringue 4 pour permettre le raccordement de l'aiguille 7 d'administration à cette dernière.

Il est à noter que le raccordement hermétique procuré par l'embout 37 de l'entrée 38 du dispositif 5 de stérilisation par filtration et par la deuxième extrémité axiale 36 de l'ajutage 26 n'est pas nécessairement démontable, l'ajutage 26 pouvant être laissé en place solidaire du dispositif 5 de stérilisation par filtration après utilisation. En effet, aussi bien l'ajutage 26 que ce dispositif 5 de stérilisation par filtration sont de préférence à usage unique, et jetés après utilisation pour la préparation d'une quantité de fluide de traitement stérile destiné à un patient.

La seringue d'administration 4 peut être par exemple une seringue jetable standard de volume compris entre 1ml et 100ml, notamment de 50ml. Cette seringue 4 comprend un piston 42 et un corps de seringue 43 définissant un cylindre 44 dans lequel le piston 42 peut coulisser librement. Le cylindre 44 débouche dans l'extrémité axiale 41 débouchante de distribution 41 du corps 43 de seringue. Dans l'exemple représenté, cette extrémité de distribution 41 forme une connexion tronconique mâle normalisée démontable à verrouillage de type «Luer-Lok®». Cette extrémité de distribution 41 est adaptée pour pouvoir recevoir l'aiguille 7 qui est elle-même dotée d'une connexion tronconique femelle à verrouillage normalisée démontable, de façon traditionnelle. L'aiguille 7 est elle-même emballée dans une poche close 45 et peut être insérée à l'intérieur du corps 43 de seringue dans un évidement de la tige de manœuvre du piston 42 comme représenté figure 1.

Dans certains modes de réalisation avantageux, le volume maximum de la cavité de la seringue 4 définie entre l'extrémité de distribution 41 et le piston 42 en position extrême à l'opposé de l'extrémité de distribution 41 est supérieur ou égal à la quantité individuelle de fluide de traitement pouvant être libérée à la pression atmosphérique (conditions standards) par le réservoir 3 lorsque la valve 10 est ouverte. Le réservoir 3 contient en effet uniquement une quantité de fluide de traitement correspondant à une quantité individuelle, destinée à une administration unique, et le cylindre de la seringue peut contenir toute cette quantité individuelle de fluide de traitement. Dans ces modes de réalisation, le volume de fluide de traitement contenu dans le réservoir 3 et pouvant être libéré par ce dernier pour une administration unique est ainsi prédéfini à la fabrication. Aucun dosage n'est donc à effectuer par l'utilisateur lors du remplissage de la seringue.

L'invention s'applique néanmoins également en variante avec un réservoir 3 contenant une quantité de fluide de traitement pouvant être libérée à la pression atmosphérique qui est supérieure à la capacité de la seringue 4, voire avec un réservoir 3 réutilisable pour la préparation de plusieurs quantités de fluide de traitement stérile, pour un même patient ou pour plusieurs patients différents. En effet, le fluide de traitement préparé dans la seringue est parfaitement stérile, et l'invention permet d'éviter toute erreur de manipulation et toute rupture d'asepsie.

Le bracelet 8 adapté pour pouvoir être placé autour du poignet du patient traité peut être doté d'une étiquette 46 qui reprend des informations d'identification du réservoir 3 de fluide de traitement utilisée. De la sorte, une « traçabilité » parfaite du fluide de traitement est assurée.

Un exemple de procédé selon l'invention d'utilisation du dispositif selon l'invention est représenté sur les figures 5 à 10, respectivement. Tout d'abord, on ouvre l'enveloppe externe 2 après avoir vérifié que les conditions de stérilisation ont bien été maintenues (l'enveloppe externe 2 peut être dotée de façon traditionnelle d'indicateurs colorés de garantie de l'état stérile). On assemble ensuite axialement le réservoir 3 à la seringue 4 préalablement dotée du dispositif 5 de stérilisation par filtration et du raccord 6, en assemblant le manchon 50 sur le bossage 52 du réservoir 3.

Il est à noter que la seringue 4 et le dispositif 5 de stérilisation, et de préférence aussi le raccord 6 (contrairement à ce qui est représenté sur les figures), sont préalablement assemblés les uns aux autres avant l'emballage dans l'enveloppe 2 et la stérilisation à l'oxyde d'éthylène de l'invention. De la sorte, pour réaliser ladite communication hermétique, il suffit de réunir le raccord 6 (déjà solidaire du dispositif 5 et de la seringue 4) et la valve 10 du réservoir 3, le manchon 50 facilitant cet assemblage, le premier tronçon 48 de la première extrémité de l'ajutage 26 pénétrant dans l'orifice 14 de la valve 10 sans ouvrir cette dernière (figure 6). L'assemblage préalable de la seringue 4 et du dispositif 5 de stérilisation permet d'éviter tout contact de la cavité interne de la seringue 4 avec l'air extérieur. On garantit ainsi que le contenu de cette cavité est toujours parfaitement stérile.

On rapproche ensuite (figure 7) axialement la seringue 4 et le réservoir 3, ce qui a pour effet de rompre les liens frangibles 53, de désolidariser l'ajutage 26 du manchon 50, de déplacer axialement la première extrémité axiale 34 de l'ajutage 26 à l'intérieur de la valve 10, de façon à réaliser entre la cavité interne de la seringue et la valve 10 du réservoir une communication de fluide isolée hermétiquement de l'extérieur, via l'ajutage 26 et le dispositif 5 de stérilisation par filtration. Dans une première course de rapprochement axial, le deuxième tronçon 49 coopère avec le joint 17 en assurant l'étanchéité. Dans une deuxième course de rapprochement axial, le premier tronçon 48 coopère avec la soupape 19 de la valve 10 pour ouvrir la valve 10 et libérer le fluide de traitement du réservoir 3 qui s'écoule dans la seringue 4, dont le piston 42 est repoussé spontanément à l'extérieur du corps de seringue 43 par la pression de fluide de traitement ainsi introduite à l'intérieur du cylindre 44. L'introduction de la quantité individuelle de fluide de traitement dans la seringue 4 se produit donc par équilibrage des pressions alors que l'on a rapproché la seringue 4 du réservoir 3. Le fluide de traitement passant dans le dispositif 5 de stérilisation par filtration est stérilisé au fur et à mesure de son introduction dans la cavité de la seringue 4.

Il est à noter que la course totale de rapprochement axial de la seringue 4 et du réservoir 3 peut être limitée par la hauteur du manchon 50 dont l'extrémité axiale 58 peut venir en butée contre le carter 39 du dispositif 5 de stérilisation par filtration. Cette course doit néanmoins être suffisante pour permettre l'ouverture de la valve 10. En position de butée, les positions relatives de la seringue 4 du réservoir 3 au cours du remplissage de la seringue sont mieux contrôlées.

On dissocie ensuite le réservoir 3 de la seringue 4. Ce faisant, le manchon 50 reste solidaire du réservoir 3 tandis que l'ajutage 26 reste solidaire du dispositif 5 de stérilisation par filtration (figures 8 et 9).

Notamment dans le cas où le fluide de traitement est un gaz, on peut alors procéder éventuellement à une dilution du gaz par de l'air atmosphérique en éjectant le surplus de gaz hors du cylindre 44 (figure 8) et en aspirant à l'intérieur de ce cylindre 44 une quantité d'air atmosphérique de dilution, via le dispositif 5 de stérilisation par filtration qui stérilise donc aussi cet air de dilution (figure 9). Ces deux étapes correspondant à la dilution ne sont pas nécessaires, notamment dans le cas où l'on utilise le fluide de traitement à l'état pur.

Après séparation du réservoir 3 de la seringue 4, la présence de l'ajutage 26, dont la première extrémité axiale 34 forme l'entrée du dispositif 5, 6 de raccordement/stérilisation et est incompatible avec l'aiguille 7 empêche tout montage accidentel ultérieur de cette aiguille 7 sur la première extrémité axiale 34 de l'ajutage 26. De préférence, l'ajutage 26 ne peut plus alors être aisément dissocié manuellement du dispositif 5 de stérilisation par filtration. Si l'ajutage 26 est cependant dissocié du dispositif 5 de stérilisation par filtration, l'embout 37 formé à l'entrée 38 de ce dispositif 5 de stérilisation par filtration empêche encore tout montage accidentel de cette l'aiguille 7 sur cette entrée 38. Il est donc nécessaire de séparer le dispositif 5 de stérilisation par filtration de la seringue 4 pour pouvoir utiliser l'aiguille 7, et la raccorder à la seringue 4.

On dissocie donc ensuite le dispositif 5 de stérilisation par filtration de la seringue 4 et on raccorde l'aiguille 7 à l'extrémité axiale 41 de la seringue. La seringue ainsi obtenue représentée figure 10 est prête à l'utilisation pour une administration au patient.

L'ensemble du dispositif selon l'invention après emballage peut être stérilisé à l'oxyde d'éthylène de façon traditionnelle, à faible coût et avec une grande fiabilité. La présence de l'opercule permet d'éviter toute pénétration d'oxyde d'éthylène à l'intérieur de la valve 10 et au contact du fluide de traitement. Le dispositif 1 se présente alors en un ensemble emballé d'un seul tenant, stérile prêt à l'emploi.

L'invention peut faire l'objet de nombreuses variantes et applications autres que celles décrites ci-dessus. En particulier, il va de soi que sauf indication contraire les différentes caractéristiques structurelles et fonctionnelles de chacun des modes de réalisation décrits ci-dessus ne doivent pas être considérées comme combinées et/ou étroitement et/ou inextricablement liées les unes aux autres, mais au contraire comme de simples juxtapositions. En outre, les caractéristiques structurelles et/ou fonctionnelles des différents modes de réalisation décrits ci-dessus peuvent faire l'objet en tout ou partie de toute juxtaposition différente ou de toute combinaison différente.

Par exemple, le réservoir 3 peut contenir uniquement un gaz de traitement sous pression (sans poche de séparation, tel qu'un gaz fluorocarboné, notamment choisi parmi CF₄, C₂F₆, C₃F₈, ou SF₆, CO₂, N₂, ou de l'air). Le raccord peut être formé uniquement de l'ajutage (sans manchon d'assemblage). L'ajutage et/ou le manchon et/ou le raccord peut être directement formé par l'entrée du dispositif 5 de stérilisation par filtration. En particulier, le dispositif de raccordement/stérilisation peut être formé d'un seul tenant indémontable interposé entre la seringue et le réservoir. Les liens frangible peuvent être remplacés par des liens uniquement déformables autorisant un déplacement axial de l'ajutage par rapport au manchon. Les différentes liaisons tronconiques peuvent être remplacées en tout ou partie par toute autre forme de liaison étanche au fluide de traitement (assemblage cylindrique collé, assemblage à joint...).

## Revendications

1. Dispositif pour la préparation extemporanée d'une quantité de fluide de traitement stérile en vue de son administration à un patient, comprenant :
- une seringue (4) dotée d'une extrémité axiale (41) débouchante formant un embout tronconique mâle,
- un dispositif (5, 6) de raccordement/stérilisation adapté pour permettre l'assemblage et le raccordement hermétique, par l'intermédiaire de ce dispositif (5, 6) de raccordement/stérilisation, de la seringue (4) à une valve de sortie d'un réservoir (3) contenant une quantité de fluide de traitement, en vue du remplissage de la seringue (4), ce dispositif (5, 6) de raccordement/stérilisation comportant :
o une sortie (40) de fluide formant un embout tronconique femelle conjugué dudit embout tronconique mâle de l'extrémité axiale (41) débouchante de la seringue (4), de sorte que ladite sortie (40) de fluide du dispositif (5, 6) de raccordement/stérilisation peut être raccordée en communication hermétique de fluide à l'extrémité axiale (41) débouchante de la seringue,
- une entrée (34) de fluide adaptée pour permettre un raccordement hermétique du dispositif (5, 6) de raccordement/stérilisation à un orifice de sortie d'un réservoir (3) contenant du fluide de traitement, ladite entrée (34) de fluide du dispositif (5, 6) de raccordement/stérilisation étant formée d'une extrémité axiale (34) débouchante d'un ajutage (26) formant un embout (26) bi-fonctionnel d'actionnement d'une valve (10) de l'orifice de sortie du réservoir et d'éjection du fluide de traitement hors de ce réservoir, ladite valve (10) du réservoir étant rappelée élastiquement axialement vers l'extérieur en position d'obturation et pouvant être repoussée axialement vers l'intérieur en vue de son ouverture pour la libération du fluide de traitement hors du réservoir,
∘ un dispositif (5) de stérilisation par filtration interposé en communication hermétique de fluide entre l'entrée et la sortie du dispositif (5, 6) de raccordement/stérilisation, ce dispositif de stérilisation par filtration pouvant être démonté de la seringue,
**caractérisé en ce que** :
- les formes de ladite entrée (34) de fluide du dispositif (5, 6) de raccordement/stérilisation sont telles que cette entrée (34) de fluide est incompatible avec tout embout tronconique femelle qui serait compatible avec l'embout tronconique mâle de l'extrémité axiale (41) débouchante de la seringue (4), de sorte que le raccordement hermétique de ladite entrée (34) de fluide du dispositif (5, 6) de raccordement/stérilisation à un tel embout tronconique femelle est impossible
- le dispositif (5, 6) de raccordement/stérilisation est choisi parmi :
∘ un dispositif (5, 6) de raccordement/stérilisation comprenant au moins un embout, dit embout proximal :
▪ interposé entre le dispositif (5) de stérilisation par filtration et ladite entrée (34) de fluide du dispositif (5, 6) de raccordement/stérilisation,
▪ adapté pour pouvoir être raccordé de façon hermétique et démontable à un embout, dit embout distal, interposé entre cet embout proximal et ladite entrée (34) de fluide du dispositif (5, 6) de raccordement/stérilisation,
▪ et dans lequel les formes de tout embout proximal du dispositif (5, 6) de raccordement/stérilisation sont telles que cet embout proximal est incompatible avec tout embout tronconique femelle qui serait compatible avec l'embout tronconique mâle de l'extrémité axiale (41) débouchante de la seringue (4), de sorte que le raccordement hermétique d'un tel embout proximal du dispositif (5, 6) de raccordement/stérilisation à un tel embout tronconique femelle est impossible,
∘ un dispositif (5, 6) de raccordement/stérilisation formé d'un seul tenant indémontable entre l'entrée et la sortie de ce dispositif (5, 6) de raccordement/stérilisation.

2. Dispositif selon selon la revendication 1 **caractérisé en ce que** le dispositif (5) de stérilisation par filtration présente une entrée formant un embout proximal du dispositif (5, 6) de raccordement/stérilisation, cet embout proximal étant adapté pour permettre le raccordement d'un embout distal d'un raccord du dispositif (5, 6) de raccordement/stérilisation, ce raccord s'étendant par rapport au dispositif (5) de stérilisation par filtration à l'opposé de la seringue, et **en ce que** cet embout proximal de l'entrée du dispositif (5) de stérilisation par filtration est incompatible avec tout embout tronconique femelle qui serait compatible avec l'embout tronconique mâle de l'extrémité axiale (41) débouchante de la seringue (4), de sorte que le raccordement hermétique d'un tel embout proximal de l'entrée du dispositif (5) de stérilisation par filtration à un tel embout tronconique femelle est impossible.

3. Dispositif selon l'une des revendications 1 ou 2 **caractérisé en ce que** le dispositif (5, 6) de raccordement/stérilisation comprend au moins un raccord (6) comprenant ledit ajutage (26) présentant une première extrémité (34) axiale débouchante formant ladite entrée du dispositif (5, 6) de raccordement/stérilisation, et une deuxième extrémité (36) axiale débouchante opposée à la première extrémité, cette deuxième extrémité formant un embout distal compatible avec une entrée (34) du dispositif (5) de stérilisation par filtration de façon à permettre un raccordement hermétique de cet embout distal de la deuxième extrémité de l'ajutage à l'entrée du dispositif (5) de stérilisation par filtration.

4. Dispositif selon les revendications 2 et 3 **caractérisé en ce que** l'embout proximal de l'entrée du dispositif de stérilisation par filtration est choisi parmi un embout au moins sensiblement cylindrique mâle et un embout au moins sensiblement cylindrique femelle, et **en ce que** l'embout distal de la deuxième extrémité de l'ajutage est choisi parmi un embout au moins sensiblement cylindrique femelle et un embout au moins sensiblement cylindrique mâle.

5. Dispositif selon l'une des revendications 3 ou 4 **caractérisé en ce que** ledit raccord (6) comprend un manchon (50) portant et entourant l'ajutage (26) et présentant une jupe (51) d'extrémité adaptée pour pouvoir coopérer avec une portée entourant un orifice de sortie d'un réservoir pour guider l'assemblage dudit raccord (6) à un tel réservoir.

6. Dispositif selon la revendication 5 **caractérisé en ce que** la jupe (51) d'extrémité est adaptée pour pouvoir entourer une portée cylindrique convexe d'un bossage (52) formant un orifice de sortie d'un réservoir.

7. Dispositif selon l'une quelconque des revendications 5 ou 6 **caractérisé en ce que** l'ajutage (26) est relié audit manchon (50) par l'intermédiaire d'au moins un lien (53) choisi parmi un lien frangible et un lien déformable, adapté pour permettre un déplacement relatif de l'ajutage (26) par rapport au manchon (50) après assemblage du manchon à un réservoir.

8. Dispositif selon l'une des revendications 1 à 7 **caractérisé en ce qu'**il comprend un réservoir (3) de fluide de traitement comprenant un orifice de sortie doté d'une valve (10) rappelée élastiquement axialement vers l'extérieur en position d'obturation et pouvant être repoussée axialement vers l'intérieur en vue de son ouverture pour la libération du fluide de traitement hors du réservoir.

9. Dispositif selon la revendication 8 **caractérisé en ce que** le dispositif (5, 6) de raccordement/stérilisation est adapté pour réaliser un assemblage de la seringue (4), de ce dispositif (5, 6) de raccordement/stérilisation, et de la valve (10) du réservoir, et pour permettre le remplissage de la seringue (4), sans fuite de fluide de traitement, par simple rapprochement de la seringue (4) et du réservoir.

10. Dispositif selon l'une quelconque des revendications 8 ou 9, **caractérisé en ce que** le réservoir (3) est une cartouche rigide.

11. Dispositif selon l'une des revendications 8 à 10, **caractérisé en ce que** le réservoir est un réservoir (3) rigide contenant une quantité de fluide de traitement sous pression telle que la quantité totale de fluide de traitement susceptible d'être libérée à pression atmosphérique par le réservoir lors de l'ouverture de la valve (10) du réservoir (3) peut être entièrement contenue dans la seringue (4).

12. Dispositif selon l'une des revendications 1 à 11, **caractérisé en ce que** le dispositif (5) de stérilisation par filtration présente une sortie formant ladite sortie du dispositif (5, 6) de raccordement/stérilisation.

13. Dispositif selon l'une des revendications 1 à 12, **caractérisé en ce qu'**il comprend une aiguille (7) stérile comprenant un embout tronconique femelle conjugué de l'embout tronconique mâle de la seringue (4) permettant le raccordement hermétique de l'aiguille à l'extrémité (41) de la seringue (4).

## Patentansprüche

1. Vorrichtung für die Zubereitung einer Menge sterilen Behandlungsfluids zum Bedarfszeitpunkt im Hinblick auf dessen Verabreichung an einen Patienten, umfassend:
- eine Spritze (4), die mit einem ausmündenden axialen Ende (41) versehen ist, das ein männliches kegelstumpfförmiges Endstück bildet,
- eine Anschluss-/Sterilisationsvorrichtung (5, 6), die dafür geeignet ist, den Zusammenbau und den hermetisch dichten Anschluss, mittels dieser Anschluss-/Sterilisationsvorrichtung (5, 6), der Spritze (4) mit einem Auslassventil eines Behälters (3), der eine Menge Behandlungsfluid enthält, im Hinblick auf das Befüllen der Spritze (4) zu ermöglichen, wobei diese Anschluss-/Sterilisationsvorrichtung (5, 6) umfasst:
-- einen Fluidauslass (40), der ein zum männlichen kegelstumpfförmigen Endstück des ausmündenden axialen Endes (41) der Spritze (4) passendes weibliches kegelstumpfförmiges Endstück bildet, sodass der Fluidauslass (40) der Anschluss-/Sterilisationsvorrichtung (5, 6) in hermetisch dichter Fluidkommunikation an das ausmündende axiale Ende (41) der Spritze angeschlossen werden kann,
- einen Fluideinlass (34), der dafür geeignet ist, einen hermetisch dichten Anschluss der Anschluss-/Sterilisationsvorrichtung (5, 6) an eine Auslassöffnung eines Behälters (3) zu ermöglichen, der Behandlungsfluid enthält, wobei der Fluideinlass (34) der Anschluss-/Sterilisationsvorrichtung (5, 6) von einem ausmündenden axialen Ende (34) eines Stutzens (26) gebildet wird, der ein zweifach funktionales Endstück (26) zum Betätigen eines Ventils (10) der Auslassöffnung des Behälters und zum Austreiben des Behandlungsfluids aus diesem Behälter bildet, wobei das Ventil (10) des Behälters elastisch axial nach außen in Verschlussstellung vorgespannt ist und im Hinblick auf sein Öffnen axial nach innen gedrückt werden kann für die Freigabe des Behandlungsfluids aus dem Behälter,
-- eine Vorrichtung (5) zur Sterilisation durch Filtration, die in hermetisch dichter Fluidkommunikation zwischen dem Einlass und dem Auslass der Anschluss-/Sterilisationsvorrichtung (5, 6) eingefügt ist, wobei diese Sterilisationsfiltrationsvorrichtung von der Spritze abgebaut werden kann,
**dadurch gekennzeichnet, dass**:
- die Formen des Fluideinlasses (34) der Anschluss-/Sterilisationsvorrichtung (5, 6) so sind, dass dieser Fluideinlass (34) mit jedem weiblichen kegelstumpfförmigen Endstück, das mit dem männlichen kegelstumpfförmigen Endstück des ausmündenden axialen Endes (41) der Spritze (4) kompatibel wäre, inkompatibel ist, sodass der hermetisch dichte Anschluss des Fluideinlasses (34) der Anschluss-/Sterilisationsvorrichtung (5, 6) an ein solches weibliches kegelstumpfförmiges Endstück unmöglich ist
- die Anschluss-/Sterilisationsvorrichtung (5, 6) ausgewählt ist aus:
-- einer Anschluss-/Sterilisationsvorrichtung (5, 6), die mindestens ein als proximales Endstück bezeichnetes Endstück umfasst:
--- das zwischen der Sterilisationsfiltrationsvorrichtung (5) und dem Fluideinlass (34) der Anschluss-/Sterilisationsvorrichtung (5, 6) eingefügt ist,
--- das dafür geeignet ist, hermetisch dicht und abbaubar an ein als distales Endstück bezeichnetes Endstück, das zwischen diesem proximalen Endstück und dem Fluideinlass (34) der Anschluss-/Sterilisationsvorrichtung (5, 6) eingefügt ist, angeschlossen werden zu können,
--- und wobei die Formen jedes proximalen Endstücks der Anschluss-/Sterilisationsvorrichtung (5, 6) so sind, dass dieses proximale Endstück mit jedem weiblichen kegelstumpfförmigen Endstück, das mit dem männlichen kegelstumpfförmigen Endstück des ausmündenden axialen Endes (41) der Spritze (4) kompatibel wäre, inkompatibel ist, sodass der hermetisch dichte Anschluss eines solchen proximalen Endstücks der Anschluss-/Sterilisationsvorrichtung (5, 6) an ein solches weibliches kegelstumpfförmiges Endstück unmöglich ist,
-- einer nicht abbaubaren, einstückig gebildeten Anschluss-/Sterilisationsvorrichtung (5, 6) zwischen dem Einlass und dem Auslass dieser Anschluss-/Sterilisationsvorrichtung (5, 6).

2. Vorrichtung nach Anspruch 1, **dadurch gekennzeichnet, dass** die Sterilisationsfiltrationsvorrichtung (5) einen Einlass aufweist, der ein proximales Endstück der Anschluss-/Sterilisationsvorrichtung (5, 6) bildet, wobei dieses proximale Endstück dafür geeignet ist, den Anschluss eines distalen Endstücks eines Anschlusses der Anschluss-/Sterilisationsvorrichtung (5, 6) zu ermöglichen, wobei sich dieser Anschluss in Bezug auf die Sterilisationsfiltrationsvorrichtung (5) der Spritze gegenüberliegend erstreckt, und dadurch, dass dieses proximale Endstück des Einlasses der Sterilisationsfiltrationsvorrichtung (5) mit jedem weiblichen kegelstumpfförmigen Endstück, das mit dem männlichen kegelstumpfförmigen Endstück des ausmündenden axialen Endes (41) der Spritze (4) kompatibel wäre, inkompatibel ist, sodass der hermetisch dichte Anschluss eines solchen proximalen Endstücks des Einlasses der Sterilisationsfiltrationsvorrichtung (5) an ein solches weibliches kegelstumpfförmiges Endstück unmöglich ist.

3. Vorrichtung nach einem der Ansprüche 1 oder 2, **dadurch gekennzeichnet, dass** die Anschluss-/Sterilisationsvorrichtung (5, 6) mindestens einen Anschluss (6) umfasst, der den Stutzen (26) umfasst, welcher ein erstes ausmündendes axiales Ende (34), das den Einlass der Anschluss-/Sterilisationsvorrichtung (5, 6) bildet, und ein zweites ausmündendes axiales Ende (36) aufweist, das dem ersten Ende gegenüberliegt, wobei dieses zweite Ende ein distales Endstück bildet, das mit einem Einlass (34) der Sterilisationsfiltrationsvorrichtung (5) kompatibel ist, um einen hermetisch dichten Anschluss dieses distalen Endstücks des zweiten Endes des Stutzens an den Einlass der Sterilisationsfiltrationsvorrichtung (5) zu ermöglichen.

4. Vorrichtung nach den Ansprüchen 2 und 3, **dadurch gekennzeichnet, dass** das proximale Endstück des Einlasses der Sterilisationsfiltrationsvorrichtung ausgewählt ist aus einem mindestens im Wesentlichen zylinderförmigen männlichen Endstück und einem mindestens im Wesentlichen zylinderförmigen weiblichen Endstück, und dadurch, dass das distale Endstück des zweiten Endes des Stutzens ausgewählt ist aus einem mindestens im Wesentlichen zylinderförmigen weiblichen Endstück und einem mindestens im Wesentlichen zylinderförmigen männlichen Endstück.

5. Vorrichtung nach einem der Ansprüche 3 oder 4, **dadurch gekennzeichnet, dass** der Anschluss (6) eine Manschette (50) umfasst, die den Stutzen (26) trägt und umschließt und eine Endschürze (51) aufweist, die dafür geeignet ist, mit einem Umfang zusammenwirken zu können, der eine Auslassöffnung eines Behälters umschließt, um den Zusammenbau des Anschlusses (6) mit einem solchen Behälter zu führen.

6. Vorrichtung nach Anspruch 5, **dadurch gekennzeichnet, dass** die Endschürze (51) dafür geeignet ist, einen zylinderförmigen konvexen Umfang einer Wulst (52), die eine Auslassöffnung eines Behälters bildet, zu umschließen.

7. Vorrichtung nach einem der Ansprüche 5 oder 6, **dadurch gekennzeichnet, dass** der Stutzen (26) mittels mindestens einer Verbindung (53) mit der Manschette (50) verbunden ist, ausgewählt aus einer brechbaren Verbindung und einer verformbaren Verbindung, die dafür geeignet ist, nach Zusammenbau der Manschette mit einem Behälter eine relative Verschiebung des Stutzens (26) in Bezug auf die Manschette (50) zu ermöglichen.

8. Vorrichtung nach einem der Ansprüche 1 bis 7, **dadurch gekennzeichnet, dass** sie einen Behandlungsfluidbehälter (3) umfasst, der eine Auslassöffnung umfasst, die mit einem Ventil (10) versehen ist, das elastisch axial nach außen in Verschlussstellung vorgespannt ist und im Hinblick auf sein Öffnen axial nach innen gedrückt werden kann für die Freigabe des Behandlungsfluids aus dem Behälter.

9. Vorrichtung nach Anspruch 8, **dadurch gekennzeichnet, dass** die Anschluss-/Sterilisationsvorrichtung (5, 6) dafür geeignet ist, einen Zusammenbau der Spritze (4), dieser Anschluss-/Sterilisationsvorrichtung (5, 6), und des Ventils (10) des Behälters auszuführen, und dafür, das Befüllen der Spritze (4) ohne Auslaufen von Behandlungsfluid durch einfaches Annähern der Spritze (4) und des Behälters zu ermöglichen.

10. Vorrichtung nach einem der Ansprüche 8 oder 9, **dadurch gekennzeichnet, dass** der Behälter (3) eine starre Kapsel ist.

11. Vorrichtung nach einem der Ansprüche 8 bis 10, **dadurch gekennzeichnet, dass** der Behälter ein starrer Behälter (3) ist, der eine solche Menge Behandlungsfluid unter Druck enthält, dass die gesamte Menge Behandlungsfluid, die beim Öffnen des Ventils (10) des Behälters (3) unter Atmosphärendruck vom Behälter freigegeben werden kann, vollständig in der Spritze (4) enthalten sein kann.

12. Vorrichtung nach einem der Ansprüche 1 bis 11, **dadurch gekennzeichnet, dass** die Sterilisationsfiltrationsvorrichtung (5) einen Auslass aufweist, der den Auslass der Anschluss-/Sterilisationsvorrichtung (5, 6) bildet.

13. Vorrichtung nach einem der Ansprüche 1 bis 12, **dadurch gekennzeichnet, dass** sie eine sterile Nadel (7) umfasst, die ein zum männlichen kegelstumpfförmigen Endstück der Spritze (4) passendes weibliches kegelstumpfförmiges Endstück umfasst, das den hermetisch dichten Anschluss der Nadel an das Ende (41) der Spritze (4) ermöglicht.

## Claims

1. Device for the extemporaneous preparation of a quantity of sterile treatment fluid, for the purpose of the administration thereof to a patient, comprising:
- a syringe (4) provided with an open axial end (41) forming a male frustoconical tip,
- a connection/sterilisation device (5, 6) adapted such that it allows the hermetically-sealed connection and assembly, via this connection/sterilisation device (5, 6), of the syringe (4) to an outlet valve of a reservoir (3) containing a quantity of treatment fluid, for the purpose of filling the syringe (4), this connection/sterilisation device (5, 6) including:
-- a fluid outlet (40) forming a female frustoconical tip paired with said male frustoconical tip of the open axial end (41) of the syringe (4), such that said fluid outlet (40) of the connection/sterilisation device (5, 6) can be connected, to form a hermetically-sealed fluid communication therebetween, to the open axial end (41) of the syringe,
- a fluid inlet (34) adapted such that it allows a hermetically-sealed connection between the connection/sterilisation device (5, 6) and an outlet orifice of a reservoir (3) containing treatment fluid, said fluid inlet (34) of the connection/sterilisation device (5, 6) being formed by an open axial end (34) of a nozzle (26) forming a bi-functional tip (26) for actuating a valve (10) of the outlet orifice of the reservoir and for ejecting treatment fluid from this reservoir, said valve (10) of the reservoir being axially and elastically displaced towards the exterior in the sealing position and capable of being axially urged towards the interior in order to open the valve so as to allow the treatment fluid to leave the reservoir,
-- a filtering sterilisation device (5) inserted such that it forms a hermetically-sealed fluid communication between the inlet and the outlet of the connection/sterilisation device (5, 6), this filtering sterilisation device being capable of being removed from the syringe,
**characterised in that**:
- the shapes of said fluid inlet (34) of the connection/sterilisation device (5, 6) are such that this fluid inlet (34) is incompatible with any female frustoconical tip that is itself compatible with the male frustoconical tip of the open axial end (41) of the syringe (4), such that the hermetically-sealed connection of said fluid inlet (34) of the connection/sterilisation device (5, 6) to such a female frustoconical tip is impossible
- the connection/sterilisation device (5, 6) is selected from the group consisting of:
-- a connection/sterilisation device (5, 6) comprising at least one so-called proximal tip:
--- inserted between the filtering sterilisation device (5) and said fluid inlet (34) of the connection/sterilisation device (5, 6),
--- adapted such that it can be connected in a hermetically-sealed and removable manner to a so-called distal tip, inserted between this proximal tip and said fluid inlet (34) of the connection/sterilisation device (5, 6),
--- and wherein the shapes of any proximal tip of the connection/sterilisation device (5, 6) are such that this proximal tip is incompatible with any female frustoconical tip that is itself compatible with the male frustoconical tip of the open axial end (41) of the syringe (4), such that the hermetically-sealed connection of such a proximal tip of the connection/sterilisation device (5, 6) to such a female frustoconical tip is impossible,
-- a connection/sterilisation device (5, 6) formed in one inseparable piece between the inlet and the outlet of this connection/sterilisation device (5, 6).

2. Device according to claim 1, **characterised in that** the filtering sterilisation device (5) has an inlet forming a proximal tip of the connection/sterilisation device (5, 6), this proximal tip being adapted such that it allows the connection of a distal tip of a connector of the connection/sterilisation device (5, 6), this connector extending relative to the filtering sterilisation device (5) opposite the syringe, and **in that** this proximal tip of the inlet of the filtering sterilisation device (5) is incompatible with any female frustoconical tip that is itself compatible with the male frustoconical tip of the open axial end (41) of the syringe (4), such that the hermetically-sealed connection of such a proximal tip of the inlet of the filtering sterilisation device (5) to such a female frustoconical tip is impossible.

3. Device according to one of claims 1 or 2, **characterised in that** the connection/sterilisation device (5, 6) comprises at least one connector (6) comprising said nozzle (26) having a first open axial end (34) forming said inlet of the connection/sterilisation device (5, 6), and a second open axial end (36) opposite the first end, this second end forming a distal tip compatible with an inlet (34) of the filtering sterilisation device (5) so as to allow a hermetically-sealed connection between this distal tip of the second end of the nozzle and the inlet of the filtering sterilisation device (5).

4. Device according to claims 2 and 3, **characterised in that** the proximal tip of the inlet of the filtering sterilisation device is selected from the group consisting of a male tip that is at least substantially cylindrical and a female tip that is at least substantially cylindrical, and **in that** the distal tip of the second end of the nozzle is selected from the group consisting of a female tip that is at least substantially cylindrical and a male tip that is at least substantially cylindrical.

5. Device according to one of claims 3 or 4, **characterised in that** said connector (6) comprises a sleeve (50) bearing and surrounding the nozzle (26) and having an end skirt (51) adapted such that it can cooperate with a surface surrounding an outlet orifice of a reservoir in order to guide the assembly of said connector (6) to such a reservoir.

6. Device according to claim 5, **characterised in that** the end skirt (51) is adapted such that it can surround a convex cylindrical surface of a boss (52) forming an outlet orifice of a reservoir.

7. Device according to any of claims 5 or 6, **characterised in that** the nozzle (26) is connected to said sleeve (50) via at least one link (53) selected from the group consisting of a frangible link and a deformable link, adapted such that it allows a relative displacement of the nozzle (26) relative to the sleeve (50) after the assembly of the sleeve to a reservoir.

8. Device according to one of claims 1 to 7, **characterised in that** it comprises a treatment fluid reservoir (3) comprising an outlet orifice provided with a valve (10) which is axially and elastically displaced towards the exterior in the sealing position and which can be axially urged towards the interior in order to open the valve so as to allow the treatment fluid to leave the reservoir.

9. Device according to claim 8, **characterised in that** the connection/sterilisation device (5, 6) is adapted such that the syringe (4), this connection/sterilisation device (5, 6), and the valve (10) of the reservoir are assembled, and such that it allows the syringe (4) to be filled, without any leak of the treatment fluid, simply by bringing the syringe (4) and the reservoir close together.

10. Device according to any of claims 8 or 9, **characterised in that** the reservoir (3) is a rigid cartridge.

11. Device according to one of claims 8 to 10, **characterised in that** the reservoir is a rigid reservoir (3) containing a quantity of pressurised treatment fluid such that the total quantity of treatment fluid that may be released at atmospheric pressure by the reservoir upon opening the valve (10) of the reservoir (3) can be contained in full in the syringe (4).

12. Device according to one of claims 1 to 11, **characterised in that** the filtering sterilisation device (5) has an outlet forming said outlet of the connection/sterilisation device (5, 6).

13. Device according to one of claims 1 to 12, **characterised in that** it comprises a sterile needle (7) comprising a female frustoconical tip paired with the male frustoconical tip of the syringe (4) allowing the hermetically-sealed connection of the needle to the end (41) of the syringe (4).
